# EUROPEAN PATENT SPECIFICATION

(11) **EP 0 242 417 B1**
(45) Date of publication and mention of the grant of the patent: **10.02.1993**
(21) Application number: 86105584.6
(22) Date of filing: 23.04.1986
(51) Int. Cl.: C07D 221/28, C07D 489/00, C07D 489/08, A61K 31/485

(54) **Morphinane-skeletoned hydrazone derivatives, process for preparing them, pharmaceutical composition and use**
Hydrazonderivate mit Morphinangerüst, Verfahren zu deren Herstellung, pharmazeutische Zusammensetzung und Verwendung
Dérivés d'hydrazone à squelette morphinane, leur procédé de préparation, composition pharmaceutique et utilisation

(43) Date of publication of application: 28.10.1987
(73) Proprietor: ALKALOIDA VEGYéSZETI GYáR, H-4440 Tiszavasvári (HU)
(72) Inventor: Hosztafi, Sandor, Dr., H-4275 Monostorpalyi (HU); Makleit, Sándor, Dr., H-4032 Debrecen (HU); Szilágyi, László, Dr., H-4032 Debrecen (HU); Zsupán, Kálmán, Dr., H-4440 Tiszavasvári (HU)
(74) Representative: Lehn, Werner, Dipl.-Ing.

(56) References cited:
- EP-A- 0 077 521
- FR-A- 1 078 467
- US-A- 2 797 221
- A. Stein: Die Pharmazie, 10, 180 (1955)
- L. Knorr, H. Hörlein: Ber., 40, 2032 (1907)
- L.J. Sargent, L.H. Schwartzman, L.F. Small, J. Org. Chem. 23 1247 (1958)
- M. Szücs et al., MTA Biol. Uszt. Közl., 25, 655 (1982)
- JOURNAL OF MEDICINAL CHEMISTRY, vol. 23, no. 6, 1980, pages 674-676; Am. Chem. Soc., US, G.W. PASTERNAK et al.: "Long-acting opiate agonists and antagonists: 14-Hydroxydihydromorphinone hydrazones."
- CHEMICAL ABSTRACTS, REGISTRY HANDBOOK; Number Section 1965-1971, pages 2629R and 2661R; Columbus, Ohio, US
- CHEMICAL ABSTRACTS, vol. 104, 1986, page 56, ref. no. 142069u; Columbus, Ohio, US; E.F. HAHN et al.: "Irreversible opiate agonists and antagonists. III. Phenylhydrazone derivatives of naloxone and oxymorphone" & J. PHARMACOL. EXP. THER. 1985, 235(3), 846-50
- CHEMICAL ABSTRACTS, vol. 103, no. 23, December 9, 1985, page 69, ref. no. 189654a; Columbus, Ohio, US; L. LI et al.: "Four long acting analgesic hydrazone derivatives of opiates which bind more firmly with opiate receptors" & ZHONGGUO YAOLI XUEBAO 1985, 6(3), 152-6
- CHEMICAL ABSTRACTS, vol. 103, no. 1, July 8, 1985, page 86, ref. no. 892n; Columbus, Ohio, US; M. SZUCS et al.: "Binding of revisible and irrevisible ligands to rat brain opiate receptors" & METHODOL. SURV. BIOCHEM. ANAL. 1984, 13(Invest. Membr.-Located Recept), 493-6
- CHEMICAL ABSTRACTS, vol. 102, no. 23, June 10, 1985, page 96, ref. no. 198310s; Columbus, Ohio, US; M. SZUCS et al.: "Binding of revisible and irrevisble ligands to rat brain opiate receptor(s)" & NEUROPEPT. PSYCHOSOM. PROCESSES, INT. CONF. INTEGR. NEUROHUMORAL MECH. 1982 (Pub. 1983) 105-10.
- CHEMICAL ABSTRACTS, vol. 100, no. 15, April 9, 1984, pages 623-624, ref. no. 121411b; Columbus, Ohio, US; M. LIU et al.: "Synethesis of long-acting analgesic hydrazone derivatives of 14-hydroxycodeinone and 14-hydroxymorphinone"& YAOXUE XUEBAO 1983, 18(6), 475-7
- JOURNAL OF THE AMERICAN CHEMICAL SOCIETY, vol. 72, July 1950, pages 3247-3249; US; S.P. FINDLAY et al.: "The preparation and properties of codeinone"
- CHEMISCHE BERICHTE, vol. 57, 1924, pages 1422-1427; E. SPEYER et al.: "über die katalytische Hydrierung von Oxy-kodeinon- und Dihydro-oxykodeinon-Hydrazon mittels Palladium-Wasserstoffs"
- JOURNAL OF ORGANIC CHEMISTRY, vol. 17, 1952, pages 1540-1544, T.D. PERRINE et al.: "Reactions of dihydrocodeinone with hydrazine and with ethyl mercaptan"
- JOURNAL OF ORGANIC CHEMISTRY, vol. 49, no. 20, 1984, pages 3824-3828; Am. Chem. Soc., US; V.M. KOLB et al.: "Syn-anti isomerism in the opiate hydrazones and azines derived from naloxone, naltrexone, and oxymorphone"
- CHEMICAL ABSTRACTS, vol. 100, no. 19, May 7, 1984, page 38, ref. no. 150886r; Columbus, Ohio, US M. SZUCS et al.: "Characterization of opiate receptors in rat brain membrane fractions" & MAGY. TUD. AKAD. BIOL. TUD. OSZT. KOZL. 1982, 25(4), 655-9

## Description

### Technical field

The present invention relates to novel morphinane-skeletoned hydrazone derivatives of the formula (I) or steric isomers thereof, 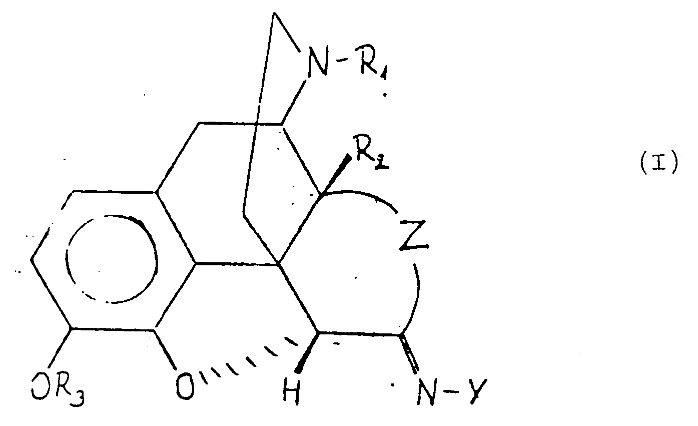
and the pharmaceutically acceptable salts thereof, a novel process for producing the same, pharmaceutical compositions comprising the same as active ingredient and their use in the therapy as analgetic or morphine-antagonistic agents.

### Background art

The reaction of 14-hydroxy dihydromorphinone, Naloxon and Naltrexon with hydrazine was reported first time in 1980 (G.W. Pasternak; E.F. Hahn: J. Med. Chem. 23 674 (1980)). When the condensation products thus formed, the hydrazones were submitted to pharmacological examinations, it turned out that the original activity of the starting molecules (analgetic or morphine-antagonistic activity) is retained, however, the duration of the effect of the compounds significantly increased
(cf. also CA, Registry Handbook, Number Section 1965-1971, pages 2629R, 5967-81-7 and page 2661R, 6047-08-1, CA 103 (23) 189654a (1985); CA 104 142069 n (1986); EP-A-0077521).

US-A-2797221 describes a process and intermediates for producing 1-bromocodeinone, including 2,4-dinitrophenylhydrazones. FR-A-1078467, refers to the total synthesis of morphine, during which synthesis 2,4-dinitrophenylhydrazones of morphinone derivatives (thebainone) have been prepared. I. Am. Chem. Soc. 72 (1950) 3247 discloses the preparation and properties of codeinone and of derivatives thereof, e.g. the 2,4-dinitrophenylhydrazone.

It was established in the course of the further examinations that the activity of the hydrazone derivatives can be attributed to the formation of ketazines [E.F. Hahn, M. Carroll-Buatti; G. Pasternak: J. Neuroscience 2 (5) 572 (1982); E.F. Hahn, G. Pasternak: Life Sci. 31 1385 (1982); S. Saletta, G.S.F. Ling, L. Wolfin, G.W. Pasternak: Life Sci. 31 1389 (1982)]. The authentic ketazines selectively link to opiate receptors in vitro 20-40 times better, than the corresponding hydrazone analogues.

When the ¹³C-NMR analysis of the hydrazones was carried out, it was found, that they are the mixture of syn and anti isomers, wherein the sterically less crowded anti isomer is the dominant [V.M. Kolb, J.R. Gober: Life Sci. 33 Suppl. I 419 (1982); V.M. Kolb; D.H. Hua: J. Org. Chem. 49 3824 (1984)]. According the above authors only the anti isomer product was formed in the reaction of oxymorphone, Naloxon, and Naltrexon with N,N-dimethyl hydrazine.

Hungarian researchers prepared Naloxone phenylhydrazine [Szücs M., Tóth G., Benyhe S.: MTA Biol. Oszt. Közl. 25 655 (1982) ,
CA 100 (19) 150886r (1984), CA 102 (23) 198310 s (1985), CA 103 (1) 892n (1985)]; in these documents naloxone phenylhydrazone is described as having a much weaker membrane affinity than naloxone.

Chinese authors described the hydrazones and N,N-dimethyl hydrazones of 14-hydroxy morphinone and 14-hydroxy codeinone [M. Liu, C. Chi, Y. Gvo, C. Zhu: Yaoxue Xuebao 18 475 (1983); C.A. 100 (15) 121411b (1984)].

As the hydrazone derivatives are very interesting from pharmacological point of view, therefore our experiments were extended to further morphine-skeletoned ketons (dihydrocodeinone, dihydromorphinone, dihydrotebainone, codeinone, 14-hydroxy codeinone, 14-hydroxy dihydrocodeinone).

In the prior art the hydrazone derivative of more morphine-skeletoned ketone has been described. They were prepared mainly for analytical purposes, as the semicarbazones, phenylhydrazones, 2,4-dinitro phenylhydrazones can be well crystallized.

The preparation of the hydrazone of 14-hydroxy codeinone and 14-hydroxy dihydrocodeinone was reported even in 1924 [E. Speyer, K. Sarre: Ber. 57 1422 (1924)].

Subject matter of the present invention are novel morphinane skeletoned compounds of formula (I) or the steric isomers thereof according to claim 1.

Preferred embodiments of these compounds are subject matter of claim 2 and 3.

Further subject matter of the present invention is a pharmaceutical composition which comprises at least one compound of formula (I), the steric isomers or the pharmaceutically acceptable salts thereof in association with at least one excipient.

Further subject matter is also a process according to claim 5 for the preparation of the compounds of formula (I) or steric isomers thereof.

Preferred embodiments of this process are subject matter of claims 6 to 9.

Further subject matter is a process for the preparation of pharmaceutical compositions according to claim 10, and the use of the compounds according to the present invention for the preparation of an analgetic or morphine-antagonistic medicament.

### Detailed description of the invention

The novel compounds of the invention are represented by the formula (I) and (I)' 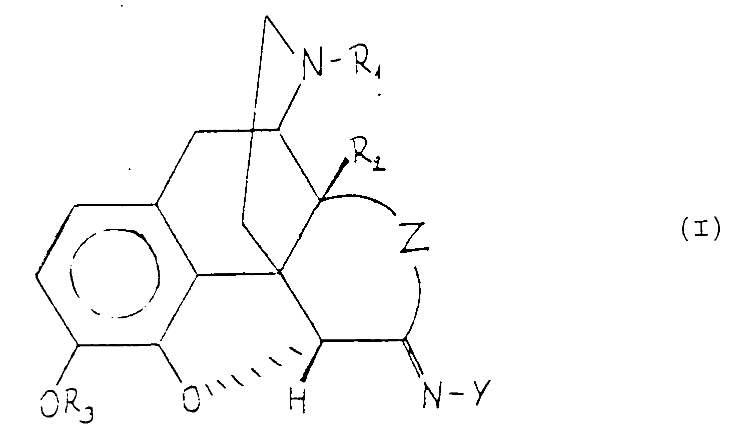
wherein Z, R₁, R₂, R₃ and Y have the following meanings:
1. Novel morphinane skeletoned compounds of formula I and I' or the steric isomers thereof 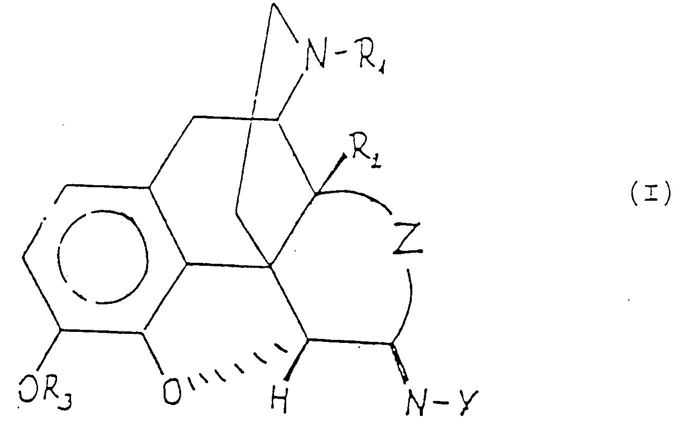 wherein
   - Z: represents-CH₂ -CH₂ or -CH=CH-,
   - R₁: stands for methyl, -CH₂CH=CH₂, 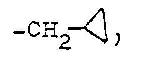
   - R₂: is hydrogen or hydroxy,
   - R₃: is hydrogen or methyl,
   - Y: stands for NH₂, -NH-CONH₂, -NH-CSNH₂, -NHC₆H₅, -NH-C₆H₃(NO₂)₂, -NHCOC₆H₅, -NH-SO₂C₆H₄-pCH₃, 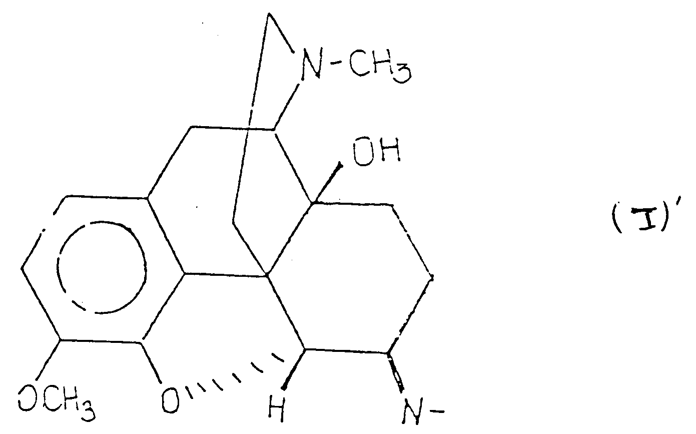
   the dotted line represents a bond or hydrogen, and the pharmaceutically acceptable salts thereof with the following provisions applying to the isomers;
when Z represents -CH₂-CH₂-, R₂ represents hydrogen,
R₃ represents methyl then Y must be different from -NH-CONH₂, -NHCOC₆H₅, -NH-C₆H₃(NO₂)₂,
when Z represents -CH=CH-, R₂ represents hydrogen and R₃ represents methyl then Y must be different from -NH-CONH₂, -NH-C₆H₃(NO₂)₂, and
when Z represents -CH₂-CH₂- or -CH=CH-, R₂ represents hydroxy, R₃ represents methyl; then Y must be different from -NHC₆H₅,
when Z is -CH₂-CH₂-, R₂ is hydrogen, R₃ is methyl and the dotted line represents a bond, Y must be different from -NH-CONH₂
with the following provisions applying to the isomer mixture:
when Z represents -CH₂-CH₂-, R₂ represents hydrogen, R₃ represents methyl then Y must be different from -NH-CONH₂, -NHCOC₆H₅, -NH-C₆H₃(NO₂)₂;
-NH₂, -NHC₆H₅, when Z represents -CH=CH-, R₂ represents hydrogen and R₃ represents methyl, then Y must be different from -NH-CONH₂, -NH-C₆H₃(NO₂)₂, and
when Z represents -CH=CH-, R₂ represents hydroxy, R₃ represents methyl, then Y must be different from -NHC₆H₅,-NH₂,-NHC₆H₄(NO₂)₂,
when Z represents-CH₂-CH₂-, R₂ represents hydroxy, R₃ represents methyl, then Y must be different from -NHC₆H₅ and -NH₂,
when Z is -CH₂-CH₂-, R₂is hydroxy, R₃ is hydrogen, the dotted line represents a bond, then Y must be different from -NH₂,
when Z represents -CH₂-CH₂-, R₂ is hydroxy, R₃ is hydrogen, the dotted line represents a bond, then Y must be different from -NH₂,
when R₁ represents methyl or -CH₂-CH=CH₂, R₂ represents hydroxy, R₃ represents hydrogen, Z is -CH₂-CH₂-, the dotted line represents a bond, then Y must be different from -NHC₆H₅,
when R₁ is methyl, R₂ is hydrogen, R₃ is methyl, Z represents -CH=CH-, and the dotted line represents hydrogen, then Y must be different from -NHC₆H₄(NO₂)₂.

The present invention also covers the stereoisomers and the pharmaceutically acceptable salts of the above compounds.

The preferred pharmaceutically acceptable salts are inorganic or organic, e.g. the salts formed with hydrochloric, sulfuric, phosphoric acid or tartaric, fumaric, malic, acetic, formic acid.

According to the invention the compounds of the formula (I), wherein
- Z: represents CH₂-CH₂ or -CH=CH-,
- R₁: stands for methyl, -CH₂CH=CH₂, 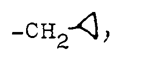
- R₂: is hydrogen or hydroxy,
- R₃: is hydrogen or methyl,
- Y: stands for NH₂, -NH-CONH₂, -NH-CSNH₂, -NHC₆H₅, -NH-C₆H₃(NO₂)₂; -NHCOC₆H₅, -NH-SO₂C₆H₄-pCH₃
and the dotted line represents a bond or hydrogen, can be prepared by reacting a-morphinane-skeletoned ketone of the formula (II) 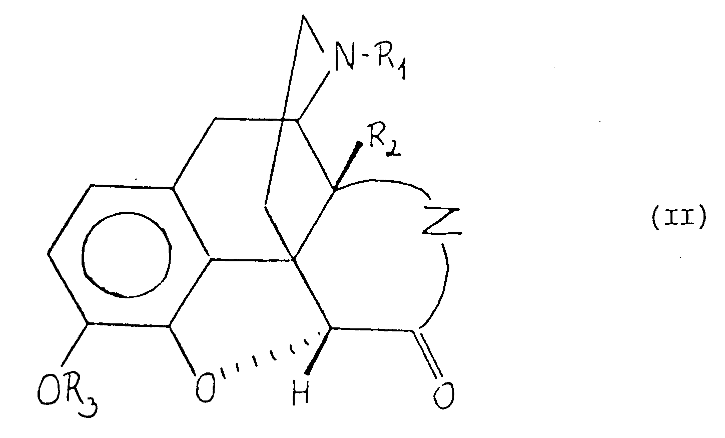
wherein Z, R₁, R₂ and R₃ are the same as defined hereinabove, the dotted line represents an optional bond, with a compound of the formula (III)

NH₂-Y (III)

wherein Y is the same as defined hereinabove.

The invention is based on the recognition that the hydrazone derivatives of the formula (I), which are the mixture of stereoisomers (syn and anti) as crude products, can be prepared in stereo-chemically uniform form by crystallization and/or chromatographic purification. The present process is suitable for the economic, industrial scale preparation of the analgetic, expectorant or morphine-antagonist morphinane derivatives being important from pharmacological and pharmaceutical points of view in stereo-chemically uniform form.

The hydrazone derivatives prepared by our process, which chemical structure was verified by modern analytical methods (IR, PMR, MS) had significantly different physical characteristics compared to the literary data. It was found that when alcohol (ethanol or methanol) or dimethyl formamide is used in the reaction as solvent, some ketazine (10 to 20 % by weight) is also formed. The structure of the ketazines was also verified besides the instrumental experiments by preparing them from hydrazone and ketone as well.

From the starting ketons codeinone [L. Knorr; H. Hörlein: Ber. 40 2032 (1907)], dihydrotebainone [J. M. Gullend; R. Robinson: J. Chem. Soc. 1923 998] and dihydrocodeinone semicarbazone [A. Stein: Pharmazie 10 180 (1955)] are known from the prior art.

Besides the above compounds the semicarbazone of dihydromorphinone, 14-hydroxy dihydromorphinone, 14-hydroxy codeinone, 14-hydroxy dihydrocodeinone, Naloxon , Naloxon methylether were also prepared.

All of the crude products proved to be composed of two components, but the pure compounds could be isolated by crystallization and/or chromatographic methods. The crude products were the mixtures of the syn and anti isomers according to PMR analysis results.

When the thiosemicarbazones were prepared, the thiosemicarbazones of dihydrocodeinone, dihydromorphinone and 14-hydroxy dihydromorphinone were obtained as a mixture of the stereoisomers, however, they could not be separated. The thiosemicarbazones of 14-hydroxy codeinone and 14-hydroxy dihydrocodeinone were obtained in stereochemically uniform form.

Besides the phenylhydrazone of dihydrocodeinone [A. Stein Pharmazie 10 180 (1955)], 14-hydroxy codeinone and 14-hydroxy dihydrocodeinone [L.J. Sargent, L.H. Schwartzman, L.f. Small: J. Org. Chem. 23 1247 (1958)] known from the prior art dihydromorphinone phenylhydrazone and 14-hydroxy dihydromorphinone phenylhydrazone were also prepared. The separation of the syn and anti isomers of these latter compounds could not be solved. The phenylhydrazone derivatives known from the prior art are stereochemically uniform compounds.

The 2,4-dinitrophenylhydrazone of 14-hydroxy codeinone and 14-hydroxy dihydrocodeinone are homogenous upon examining by thin-layer chromatography while they are stereochemically uniform on the basis of the PMR spectra. When dihydromorphinone and 14-hydroxy dihydromorphinone were used as starting materials always the mixture of the syn and anti isomers could be obtained even the reaction conditions were changed, and these isomers could not be separated.

The dihydrotebainone-2,4-dinitrophenyl hydrazone is uniform on the basis of thin-layer chromatographic analysis, however, the PMR spectra does not form a ground for the decision whether it is the syn or anti isomer.

In order to analyse the PMR spectra, the prior art 2,4-dinitrophenyl hydrazone of codeinone [S.P. Findlay; L.F. Small: J. Am. Chem. Soc. 72 3247 (1950)] and dihydrocodeinone [A. Stain: Pharmazie 10 180 (1955)] were also prepared.

The activity of the compounds of the invention was examined as follows.

The effect of the 10⁻⁷ and 10⁻⁵ M concentration of the compounds was examined to tritiated Naloxon bond in rat brain membrane preparates. The results show how the compounds are able to compete for the specific binding positions of the labelled Naloxon. Table I summarizes the measure of the residual tritiated specific bonds of Naloxon.

If the inhibition is high, i.e. the value of the specific bond of the labelled Naloxon is low, the compounds bind to the opiate receptors with high affinity. Those compounds which decreased the specific bond only in low extent (even in a concentration of 10⁻⁵) cannot be considered as significantly bound to the opiate receptors.

A comparative data the tritiated Naloxon bond (concentration: 1 nanomole) was taken as 100 % (without excipients).

**Table I**

| Compound | Concentration | |
|---|---|---|
| | 10⁻⁷ mol | 10⁻⁵ mol |
| Oxycodeinone hydrazone | 92±25 | 13±7 |
| Dihydrocodeinone semicarbazone | 43±4 | 9±7 |
| Dihydromorphinone semicarbazone | 69±13 | 4±6 |
| Dihydrocodeinone phenylhydrazone | 75±9 | 5±1 |
| Dihydromorphinone phenylhydrazone | 4±5 | 10±14 |
| Dihydromorphinone dinitrophenylhydrazone | 17±2 | 0.5±0.7 |
| Oxymorphone dinitrophenylhydrazone | 33±1 | 1±0.3 |
| Dihydromorphinone thiosemicarbazone | 16±5 | 19±4 |
| Oxymorphone thiosemicarbazone | 37±9 | 0 |

The membrane preparate was prepared according to the following article: G.W. Pasternak, H.A. Wilson, S.H. Snyder: Molecular Pharmacol. 11 340-51 (1975).

The specific radioactivity of the labelled Naloxon was 72.4 Ci/mmole [G. Töth, M. Kramer, F. Sirokmán, A. Borsodi, A. Rónai: J. Label. Comp. Radiopharm. 19 1021 (1976)].

The analgetic activity of the compounds of the invention is summarized in Table II.

**Table II**

| The analgetic activity of the compounds of the invention | | |
|---|---|---|
| | Hot plate test ED₅₀ mg/kg.sc | Tail Flick ED₅₀ mg/kg.sc. |
| Dihydromorphinone semicarbazone | 0.085 | 0.13 |
| Oxycodone semicarbazone | 0.58 | 0.45 |
| Dihydroceinone semicarbazone | 0.38 | 0.35 |
| Dihydrocodeinone thiosemicarbazone | 1.7 | 0.6 |
| Oxycodone phenylhydrazone | 0.8 | 1.2 |
| Dihydrocodeinone phenylhydrazone | 2.4 | 2.3 |
| Oxymorphone dinitrophenylhydrazone | 0.37 | 0.27 |
| Dihydromorphinone dinitrophenylhydrazone | 0.27 | 0.18 |
| Oxymorphone semicarbazone | 0.9 | 0.9 |
| Morphine | 3.6 | 5.0 |

The compounds wherein a methyl group is bound to the nitrogen are effective analgetic agents, they also have cataleptogenic activity, further potentiorate the effect of barbiturates.

The compounds wherein an allyl or cyclopropylmethyl group is bound to the nitrogen, exhibit morphine antagonistic activity.

The invention is illustrated by the following, non-limiting examples.

For the thin-layer chromatographic tests Merck 5554 silica gel 60 F₂₅₄ foils were used. As eluents the following mixtures were applied:
- A:: chloroform/acetone/diethyl amine = 5:4:1
- B:: chloroform/methanol/cc. ammonium hydroxide = 90:10:5
- C:: chloroform/methanol = 9:1
- D:: benzene/methanol = 8:2
The spots were detected in UV light and by Draggendorff reagent.

The PMR spectras were taken in a Bruker W200SY instrument, the chemical shift is given in ppm.

When the steric isomers are characterized, the trans (E) and cis (z) isomer is given compared to the 5β protone.

The mass spectras were taken on a VG-7035 (GC-MS-DS) instrument. The relative intensities are indicated after the mass numbers in parenthesis.

### Example 1

### 14-OH-dihydrocodeinione hydrazone

5 ml of 100 % hydrazine hydrate are dissolved in 10 ml of dimethyl formamide, then 2.0 g of 14-OH-dihydrocodeinone are added in small portions to the above solution under stirring. The solution is kept over a water bath for 2 hours, then poured into 100 ml of ice water after cooling. The precipitated crystalline substance is filtered off, washed with cold water. 1.5 g of crude product are obtained. The pure fractions obtained after chromatographing on silica gel (eluent: a 9:1 mixture of chloroform and ethanol) (further eluent B) are recrystallized from methanol. M.p.: 192-4 ^{o}C.
PMR (CDCl₃): 6.6; 6.7 dd (H-1.2; 2H), 5.33 s (NH₂; 2H deuterable), 4.93s (H-5β; 1H), 3.8s (OCH₃; 3H), 2.35s (N-CH₃; 3H)
MS (C₁₈H₂₃N₃O₃; 329.38) 329(92) 313(10)

### Example 2

### 14-OH-dihydrocodeinone azine

0.6 g of 14-OH-dihydrocodeinone hydrazone and 0.7 g of 14-OH-dihydrocodeinone are dissolved in 30 ml of dry benzene and refluxed for 4 hours. According to thin-layer chromatographic examinations (using eluent B) the hydrazone has transformed, a new spot can be observed besides the spot of the ketone used in an excess. After evaporation off of the benzene, the residue is recrystallized from ethanol twice. 0.25 g of azine are obtained with a melting point of 199-202 ^{o}C.
PMR (CDCl₃): 6.65 d (H-1.2; 2H), 4.95s (H-5; 1H), 3.75s (OCH₃; 3H), 2.35s (N-Me; 3H)
MS (C₃₆H₄₂N₄O₆; 626.73) 626(8) 611(7) 313(43)

### Example 3

### Dihydrocodeinone hydrazone

4 ml of dry hydrazine are dissolved in 20 ml of dry methanol and 1.0 g of dihydrocodeinone are added in small portions under stirring. After two hours stirring the methanol is evaporated off under vacuo, 50 ml of sodium tetraborate are added to the residue and the solution is extracted with 3x25 ml of chloroform. The chloroform phase is washed with saline and dried over magnesium sulfate. 1.0 g of oily hydrazone comprising a very small amount of azine determined by thin-layer chromatography (using eluent B) is obtained.

Pure hydrazone can be achieved by preparative thin-layer chromatography, but this substance also is a mixture of stereoisomers.
PMR (CDCl₃): 6.7dd (H-1.2; 2H), 6.3s (NH₂; 1H trans), 5.25 s (H-5β and NH₂; 2H cis), 4.9s (H-5β; 1H; trans), 3.8d (OCH₃), 2.35s (N-CH₃; 3H), The cis/trans ratio is 67/33.
MS (C₁₈H₂₃N₃O₂ - 313.38) 313(40) 298(11)

### Example 4

### Dihydromorphinone hydrazone

0.90 g of dihydromorphinone hydrazone are prepared from 1.0 g of dihydromorphinone according to Example 3. The product is a colourless oil, it comprises about 15 to 20 % of ketazine according to PMR and TLC experiments. Even the substance purified by preparative thin-layer chromatography does not crystallize and it is a mixture of stereoisomers according to the PMR spectra.
PMR (CDCl₃): 6.6-6.7m (H-1.2; cis + trans), 5.3s (NH₂ + OH), 5.23s (H-5β; 1H cis), 4.85s (H-5β; 1H trans), 2.4s (N-Me; 3H). The cis/trans ratio is 30:70.

### Example 5

### 14-OH-codeinone hydrazone

According to the method described in Example 1 1.8 g of 14-OH-codeinone hydrazone were obtained using 2.0 g of 14-OH-codeinone as starting material. Upon examining the endproduct by thin-layer chromatography, two spots were observed. The pure main component can be obtained by preparative thin-layer chromatographic method. The melting point of the title compound is 212-15 ^{o}C after recrystallization from ethanol.
PMR (CDCl₃): 6.7dd (H-1.2; 2H), 6.3m (H-7.8; 2H), 5.65d (NH₂; 2H deuterable) 5.38s (H-5β; 1H), 3.75s (OCH₃; 3H), 2.4s (N-Me; 3H)
MS (C₁₈H₂₁N₃O₃ - 327.37) 327(52) 309(17) 281(41)

### Example 6

### Dihydrocodeinone semicarbazone

In 50 ml of water 1.25 g of semicarbazide chlorohydrate are dissolved, thereafter 3.0 g of dihydrocodeinone (base) are added. The mixture is heated on a water bath for 30 minutes, thereafter it is made alkaline with concentrated sodium carbonate solution (pH ∼ 9-10) after cooling to a temperature of +5 ^{o}C. The precipitated crystals are filtered, washed with cold water. 3.0 g of crude product are obtained. This crude product comprises two components according to thin-layer chromatographic examination (with eluent mixtures A and B), which are the syn and anti isomers of the title product. After recrystallization from propanol or aqueous ethanol chromatographically homogenous product can be obtained. M.p.: 244-247 ^{o}C.
PMR (CDCl₃): 9.65s (NH; 1H), 7.8s (NH₂; 2H), 6,7dd (H-1.2; 2H), 4.92s (H-5β; 1H), 3.85s (OCH₃; 3H), 2.4s (N-CH; 3H). The 5-β-H shifts in the crude product comprising 50-50 % syn and anti isomers are as follows: 5.2 (cys) and 4.92 (trans) ppm.
MS (C₁₉H₂₄N₄O₃ - 356.41) 356(51) 339(38) 297(100) 284(37)

### Example 7

### 14-OH-dihydrocodeinonesemicarbazone

The method of Example 6 was followed except that 3.0 g of 14-OH-dihydrocodeinone were used as starting material. 3.2 g of crude product were obtained, which is a mixture of the syn and anti isomers according to thin-layer chromatographic examinations. After recrystallization from a mixture of chloroform and ethanol, pure semicarbazone can be achieved. M.p.: 236-238 ^{o}C.
PMR (DMSO-d₆): 9.25s (NH; 1H), 6.8dd (H-1.2; 2H), 6.4s (NH₂; 2H), 4.81s (H-5β; 1H), 3.75s (OCH₃; 3H), 2.3s (N-Me; 3H). The 5-β-H shifts in the crude product comprising the isomers in a weight ratio of 1:1 are as follows: 4.85 (trans) and 5.17 (cys) ppm (CDCl₃).
MS (C₁₉H₂₄N₄O₄ - 372.41) 372(25) 355(58) 313(70)

### Example 8

### 14-OH-codeinone-semicarbazone

The method of the preceeding example was followed except that 3.0 g 14-OH-codeinone were used as starting material. 2.8 g of crystalline crude product are formed, which is a mixture of the syn and anti isomers according to thin-layer chromatographic examinations. After recrystallization from methanol, a uniform product can be achieved.
M.p.: 260-263 ^{o}C (dec.)
PMR (CDCl₃): 8.72s (NH; 1H), 6.7dd (H-1.2; 2H), 6.3d (H-8; 1H), 5.85d (H-7; 1H), 5.19s (H-6β; 1H; trans), 5.0s (NH₂; 2H 3.8s (OCH₃; 3H), 2.4s (N-CH₃; 3H)
MS (C₁₉H₂₂N₄O₄ - 370.40) 370(35) 353 (22) 313(29)

### Example 9

### Codeinone semicarbazone

1.2 g semicarbazide chlorohydrate are dissolved in 50 ml of water and the solution is heated on a water bath for a hour after the addition of 3.0 g of codeinone. After cooling to +5 ^{o}C, the solution is alkalified by concentrated sodium carbonate solution (pH∼9-10), and the separated oil is extracted with 3x30 ml of chloraform. The chloroform phase is washed with saline, dried over magnesium sulfate and evaporated off. The residue is crystallized from ethanol and small chloroform. The melting point of codeinon semicarbazone (1.3 g) is the same as referred to in the prior art [Knorr, L., Hörlein, H.: Ber. 40 2032 (1407)], but it gives two spots on a chromatoplate According to the PMR spectra the product is the mixture of the syn and anti isomers.
PMR (CDCl₃): 8.75s (NH; 1H), 6.7m (H-1.2; 2H), 6.2dd (H-8; 1H), 5.85dd (H-7; 1H), 5.2s (H-5β; 1H; trans), 3.8s; 3.81s (OCH₃; 3H), 2.4s (N-Me; 3H)
MS (C₁₉H₂₂N₄O₃ - 354.40) 354(100) 337(23)

### Example 10

### 14-OH-dihydromarphinone semicarbazone

The procedure of Example 6 was followed except that the reaction was carried out within 2 hours. 2.5 g of semicarbazone were obtained from 3.0 g of 14-OH-dihydromorphinone, which proved to be homogenous upon examining by thin-layer chromatography. The melting point of the product is 217-220 ^{o}C after recrystallization from ethanol.
PMR (CDCl₃): 8.35s (NH; 1H), 6.6dd (H-1.2; 2H), 6.0s (NH₂; 2H), 4.93s (H-5β; 14; trans), 2.3s (N-Me; 3H)
MS (C₁₈H₂₂N₄O₄ - 358.39) 358(5) 327(49)

### Example 11

### Dihydromorphinone semicarbazone

The procedure of Example 10 was followed except that 2.85 g of dihydromorphinone were used as starting material. 3.2 g of dihydromorphinone semicarbazone were obtained, which gave more spots upon examining by thin-layer chromatography (eluent: eluent systems A and B). The minor impurity component could be removed by recrystallization from methanol and a homogenous product could be achieved. M.p.: 195-198 ^{o}C.
PMR (DMSO-d₆): 6.6dd (H-1.2; 2H); 6.4s (NH₂; 2H), 4.9s (H-5β; 1H), 2.3s (N-Me; 3H),
The 5-β protons' shifts in the crude product comprising the trans and cys isomer in a weight ratio of 71:29 are as follows: 4.9 (trans) and 5.34 (cys) ppm.
MS (C₁₈H₂₂N₄O₃ - 342.39) 342(58) 325(85) 283(86)

### Example 12

### Naloxon semicarbazone

The procedure of Example 10 was followed except that 0.65 g of Naloxon and 0.25 g of semicarbazide chlorohydrate were used as starting materials. 0.5 g of Nalaxon semicarbazone were formed, which proved to be homogenous upon examining by thin-layer chromatography. M.p.: 239-242 ^{o}C (dec.)
PMR (DMSO-d₆): 9.15s (NH; 1H), 6.45dd (H-1.2; 2H), 5.8m (allyl-proton; 1H), 5.0-5.2m (allyl-proton; 2H), 4.75s (H-5β; 1H)
MS (C₂₀H₂₄N₄O₄ - 384.42) 384(6) 298(12)

### Example 13

### Naloxon methylether semicarbazone

In 15 ml of water 0.25 g of semicarbazide chlorohydrate are dissolved and 0.68 g of Naloxon-methylether are added, then the solution is heated on a water bath for two hours. After cooling the solution is made alkaline by adding a concentrated sodium carbonate solution (pH ∼ 9), the crystalline substance is filtered off, and washed with water. 0.6 g of crude product are obtained, which gives two spots upon examining by thin-layer chromatography. The two spots are the syn and anti isomers.
PMR (DMSO-d₆): The OCH₃ group has two signs (3.75 ppm).
The shifts of the 5β protons are as follows: 4.70 (trans) and 4.82 (cys) ppm. Weight ratio of the isomers:21:79.
MS (C₂₁H₂₆N₄O₄ - 398.45)

### Example 14

### Dihydrotebainone semicarbazone

The procedure of Example 6 was followed except that 3.0 g of dihydrotebainone were used as starting material 2.7 g of crude dihydrotebainone semicarbazone are obtained which are homogenous upon examining by thin-layer chromatography. The melting point of the product is 226-227 ^{o}C after recrystallization from ethanol.
PMR (CDCl₃): 8.48s (NH; 1H), 6.65dd (H-1.2; 2H), 6.15s (NH₂; 2H), 3.8s (OCH₃; 3H), 2.4s (N-Me; 3H)
MS (C₁₉H₂₆N₄O₃ - 358.43) 358(42) 341(23) 299(100)

### Example 15

### Dihydrocodeinone thiosemicarbazone

3.0 g of dihydrocodeinone are suspended in 15 ml of water, thereafter it is dissolved with the aid of 10 ml of 1N hydrochloric acid. 1.0 g of thiosemicarbazide is added, and the solution is heated on a water bath for two hours. The hot solution is filtered off and the dihydrocodeinon thiosemicarbazon hydrochloride salt (4.0 g) quickly precipitates. This salt is recrystallized from water. M.p.: at 210 ^{o}C the product becomes yellow and decomposes between 250-255 ^{o}C. The hydrochloric salt is dissolved in water (1.0 g in 100 ml of water) and the base is liberated by adding a concentrated sodium carbonate solution (pH∼9). This product is filtered and washed with water. The product is a mixture of steric isomers according to the PMR spectra.
PMR (DMSO-d₆): 10.32s (NH; 2H cis and trans) 8.32s (NH₂; 2H cis and trans), 7.75s (NH₂; 1H cis), 7.45s (NH₂; 1H trans), 6.6-6.75m (H-1.2), 5.45s (H-5β; 1Hc); 4.87s (H-5β; 1Ht), the isomer ratio is 63:37 = = trans:cis.
MS (C₁₉H₂₄N₄O₂S - 372(29) 355(17) 297(20) 242(48)

### Example 16

### 14-OH-dihydrocodeinone thiosemicarbazone

3.1 g of 14-OH dihydrocodeinone are transformed into 4.2 g of 14-OH-dihydrocodeinone thiosemicarbazone hydrochloride according to the method of Example 15.

The hydrochloride salt well crystallizes from water, its melting point (capillary) is 230-235 ^{o}C (decomp.).

The melting point of the base is 150-154 ^{o}C (decomp.).
PMR (CDCl₃): 8.63s (NH; 1H), 7.48s (NH₂; 1H), 6.65dd (H-1.2; 2H), 6.4s (NH₂; 1H), 4.92s (H-5β; 1H), 3.8s (OCH₃; 3H), 2.35s (N-Me; 3H). Pure trans isomer.
MS (C₁₉H₂₄N₄O₃S - 388.47) 388)72) 371(100) 313 (42)

### Example 17

### 14-OH-codeinone thiosemicarbazone

According to the method described in Example 15 3.7 g of 14-OH-codeinone-thiosemicarbazone hydrochloride are prepared from 3.1 g of 14-OH-codeinone. After recrystallization from water it melts at 290-295 ^{o}C (decomposition).

The melting point of the base is 233-234 ^{o}C (dec.).
PMR (DMSO-d₆): 10.15s (NH; 1H), 8.48s; 7.95s (NH₂; 2H), 6.6dd (H-1.2; 2H), 6.25d (H-7; 1H), 6.05s (H-8; 1H), 5.87 (H-5β; 1H), 3.7s (OCH₃; 3H), 2.35s (N-CH₃; 3H),
MS (C₁₉H₂₂N₄O₃S - 386.46): 386(100); 369(50) 311(43)

### Example 18

### Dihydromorphinone thiosemicarbazone

According to the method described in Example 15, 2.5 g of dihydromorphinone thiosemicarbazone hydrochloride are prepared from 2.8 g of dihydromorphinone. Though the endproduct well crystallizes from water, it does not have a defined melting point.

After several recrystallization of the hydrochloric salt, the base is prepared. On the basis of thin-layer chromatographic examinations (eluent: A or B solvent mixture) the base comprises two components.
PMR (DMSO-d₆): 10.41s (NH; 1H cis), 10.32s (NH; 1H trans), 9.10s (C₃-OH), 8.25s (NH₂; 2H cis and trans), 7.67s (NH₂; 1H cis), 7.52s (NH₂; 1H trans) 6.55 s (H-1.2; 2H), 5.45s (H-5β; 1H cis), 4.85s (H-5β; 1H trans) 2.3s (N-Me; 3H) isomer ratio is 69:31 = = trans:cis.
MS (C₁₈H₂₂N₄O₂S - 358.45)

### Example 19

### 14-OH dihydromorphine thiosemicarbazone

According to the method of Example 15 2.8 g of 14-OH-dihydromorphinone thiosemicarbazone hydrochloride are prepared from 3.0 g of 14-OH-dihydromorphine. The hydrochloride salt does not have a defined melting point even after several recrystallization from water. The base comprises two components according to thin-layer chromatographic analysis. The base well crystallizes from ethanol or methanol, but it is not homogenous upon examining by thin-layer chromatography.
PMR (DMSO-d₆): 10.18s (NH; 2H cis + trans) 8.32s (NH₂; 2H; cis + trans) 7.65s (NH₂; 1H cis) 7.55s (NH₂; 1H trans) 5.33s (H-5β; 1H cis), 4.76s (H-5β; 1H trans), 2.3s (N-Me; 3H) isomer ratio: cis/trans 21/79.
MS (C₁₈H₂₂N₄O₃S - 374.45)

### Example 20

### Dihydrocodeinone phenylhydrazone

1.5 g of dihydrocodeinone are dissolved in 30 ml of dry ethanol, then 1.5 ml of freshly distilled phenylhydrazine and 1.0 ml of glacial acetic acid are added. The solution is boiled on a water-bath for 30 minutes, thereafter cooled and made alkaline by concentrated ammonium hydroxide. The product quickly crystallizes.
PMR (CDCl₃): 7.3-6.8m (C₆H₅; 5H), 6.7dd (H 1.2; 2H), 5.13s (H-5β; 1H), 3.8s (OCH₃; 3H), 2.35s (N-Me; 3H), pure trans isomer.
MS (C₂₄H₂₇N₃O₂ - 389.48) 389(90) 252(8) 212(15)

### Example 21

### 14-OH-dihydrocodeinone phenylhydrazone

Using 1.55 g of 14-OH-dihydrocodeinone as starting material 1.7 g 14-OH-dihydrocodeinone phenylhydrazone were prepared according to the procedure used for the preparation of dihydrocodeinone phenylhydrazone. After recrystallization from ethanol, the product melts at 174-176 ^{o}C.
PMR (CDCl₃): 9.5s (NH; 1H), 7.3-7.0m (C₆H₅; 5H), 6.7dd (H-1.2; 2H), 5.37s (H-5β; 1H), 4.9s (OH; 1H), 3.75s (OCH₃; 3H), 2.4s (N-Me; 3H), pure cys isomer.
MS (C₂₄H₂₇N₃O₃ - 405.48) 405(100) 386(7) 228(17)

### Example 22

### 14-OH-codeinone phenylhydrazone

Using 1.55 g of 14-OH-codeinone as starting material 1.4 g of 14-OH-codeinone phenylhydrazone was prepared according to the method of Example 20. The product well crystallizes from ethanol. M.p.: 192-194 ^{o}C. The product is sensitive to light, it turns to yellow due to the effect of light.
PMR (CDCl₃): 8.75s (NH; 1H), 7.3-6.9m (C₆H₅; 5H), 6.7dd (H-1.2; 2H), 6.45d (H-8; 1H), 5.7d (H-7; 1H), 5.42s (H-5β; 1H cis), 3.75s (OCH₃; 1H), 2.4s (N-Me; 3H)
MS (C₂₄H₂₅N₃O₃ - 403.46) 403(8) 385(77) 370 (13)

### Example 23

### Dihydromorphinone phenylhydrazone

1.4 g of dihydromorphinone and 1.3 ml of freshly distilled phenylhydrazone are dissolved in 30 ml of dry ethanol, thereafter refluxed for 30 minutes. The ethanol is distilled off, then the residual red oil is triturated with ether. The crystalline substance thus obtained is filtered off, washed with ether and small cold ethanol. 1.0 g of dihydromorphinone phenylhydrazone is obtained which is composed of two components according to thin-layer chromatographic examinations (eluent: A or B eluent mixture). According to PMR analysis the two components are the syn and anti isomers (trans/cis = 28/72).
PMR (DMSO-d₆): 9.67s (NH; 1H cis), 8.82s (NH; 1H trans) 7.3-7.0m (C₆H₅; 5H), 6.6-6.7m (H-1.2), 5.4s (H-5β; 1H; cis), 5.12s (H-5β; 1H; trans), 2.4s (N-Me; 3H),
MS (C₂₃H₂₅N₃O₂ - 375.45) 375(100) 283(19) 212(20)

### Example 24

### 14-OH-dihydromorphinone phenylhydrazone

1.2 g of 14-OH-dihydromorphinone phenylhydrazone were prepared according to the method of Example 23 starting from 1.5 g of 14-OH-dihydromorphinone. The product is composed of two components, they proved to be the syn and anti isomers.
PMR (CDCl₃): 9.3s (NH; 1H), 7.2-7.0m (C₆H₅), 6.7-6.5m (H-1.2; 2H), 5.3s (H-5β; 1H; cis) 5.05s (H-5β; 1H; trans) 2.3s (N-Me; 3H),
MS (C₂₃H₂₅N₃O₃ - 391.45) 391(100)

### Example 25

### Dihydrocodeinone-2,4-dinitro-phenyl-hydrazone

1.0 g of 2,4-dinitrophenyl hydrazine is dissolved in 40 ml of concentrated hydrochloric acid, thereafter 200 ml of water and 1.5 g of dihydrocodeinone are added (hot). The mixture is slightly boiled for 5 minutes, thereafter left to cool. The yellow dihydrocodeinone-2,4-dinitrophenyl hydrazone hydrochloride quickly crystallizes. The weight of the product is 2.3 g after filtration and washing with cold water. The base can be precipitated from the diluted aqueous solution of the hydrochloric salt with the aid of concentrated sodium carbonate solution (pH∼9). The melting point of the base is 207-208 ^{o}C after crystallization from ethanol or n-propanol.
PMR (CDCl₃): 12.85s (NH; 1H), 9.1d (Ar-H; 1H), 8.25dd (Ar-H; 1H), 7.9d (Ar-H; 1H), 6.7dd (H-1.2; 2H), 5.32s (H-5β; 1H cis), 3.75s (OCH₃; 3H), 2.4s (N-Me; 3H)
MS (C₂₄H₂₅N₅O₆ - 479.48) 479(100) 462(M-17; 20)

### Example 26

### 14-OH-dihydrocodeinone-2,4-dinitrophenyl hydrazone

The method of the preceeding example is followed. 2.1 g of 14-OH-dihydrocodeinone-2,4-dinitrophenyl hydrazone hydrochloride are obtained from 1.55 g of 14-OH-dihydrocodeinone. The base can be crystallized from a mixture of chloroform and ethanol. M.p.: 232-233 ^{o}C.
PMR (CDCl₃): 12.8s (NH; 1H), 9.15d (Ar-H; 1H), 8.33dd (Ar-H; 1H), 7.9d (Ar-H; 1H), 6.75dd (H-1.2; 2H), 5.33s (H-5β; 1H cis), 3.8s (OCH₃; 3H), 2.4s (N-Me; 3H),
Ms (C₂₄H₂₅N₅O₇ - 49.548) 495(40) 478(M-17; 16)

### Example 27

### 14-OH-codeinone-2,4-dinitrophenyl hydrazone

The method of Example 25 is followed except that 1.55 g of 14-OH-codeinone is used as starting material. 2.2 g of 14-OH-codeinone-2,4-dinitrophenyl hydrazone hydrochloride are obtained. The base liberated from the salt crystallizes from ethyl acetate, mp.: 157-160 ^{o}C.
PMR (CDCl₃): 12.15s (NH; 1H), 9.2d (Ar-H; 1H), 8.44dd (Ar-H; 1H), 7.95d (Ar-H; 1H), 6.7dd (H-1.2; 2H), 6.5d (H-8; 1H), 6.1d (H-7; 1H), 5.43s (H-5β; 1H cis), 3.8s (OCH₃; 3H), 2.4s (N-Me; 3H)
MS (C₂₄H₂₃N₅O₇ - 493.46) 493(27) 476(M-17; 10)

### Example 28

### Codeinone-2,4-dihydrophenyl hydrazone

1.2 g of codeinone and 0.9 g of 2,4-dinitrophenyl hydrazine are stirred in 20 ml of 96 % of acetic acid on a water bath for 30 minutes. The mixture is cooled (0 - 5 ^{o}C) and alkalified by concentrated ammonium hydroxide solution (pH 9). The precipitated oil is extracted with 3x25 ml of chloroform. The chloroformic solution is washed with brine and dried over magnesium sulfate. After evaporation of the solvent the yellow crystalline residue (1.3 g) is crystallized from a mixture of ethanol and chloroform. Weight: 0.75 g M.p.: 259-261 ^{o}C.
PMR (CDCl₃): 12.18s (NH; 1H), 9.2d (AR-H; 1H), 8.4dd (Ar-H; 1H), 7.95d (Ar-H; 1H), 6.7dd (H-1.2; 2H), 6.35dd (H- ; 1H), 6.05dd (H- ; 1H), 5.43s (H-5β; 1H cis), 3.8s (OCH₃; 3H), 2.3s (N-Me; 3H)
MS (C₂₄H₂₃N₅O₆ - 477.46) 477(100) 460 (M-17; 8)

### Example 29

### Dihydromorphinone-2,4-dinitrophenyl hydrazone

According to the method of Example 25 2.0 g of dihydromorphinone-2,4-dinitrophenyl hydrazone hydrochloride are prepared from 1.4 g of dihydromorphinone. The base liberated from the salt is composed of two components, it is the mixture of the syn and anti isomers according to thin-layer chromatographic analysis (eluent: A and B eluent system).

The two isomers cannot be separated either by crystallization (ethanol or methanol) or chromatographically (column chromatography or preparative thin-layer chromatography). The recrystallization of the hydrochloride from water does also not give the desired result.
PMR (CDCl₃): 13.17s (NH; 1H;cis), 11.10s (NH; 1H, trans) 9.15dd (Ar-H; 1H), 8.25dd (Ar-H; 1H), 6.7m (H-1.2; 2H), 5.35s (H-5β; 1H cis), 5.07s (H-5β; 1H trans), 2.4s (N-Me; 3H) cis-trans ratio 27:73.
MS (C₂₃H₂₃N₅O₆ - 465.35) 465(63) 448(M-17; 12) 431(M-34; 34)

### Example 30

### 14-OH-dihydromorphinone-2,4-dinitrophenyl hydrazone

According to the method of Example 25 1.5 g of 14-OH-dihydromorphinone-2,4-dinitrophenylhydrazone hydrochloride are prepared from 1.5 g of 14-OH-dihydromorphinone.

The precipitated base is composed of two components according to thin-layer chromatographic analysis.

The two isomers (syn and anti) cannot be separated either by crystallization or preparative thin-layer chromatography.
PMR (CDCl₃): 13.1s (NH; 1H trans), 11.15s (NH; 1H cis) 9.2m (Ar-H), 8.3m (Ar-H), 8.1d and 7.9d (Ar-H), 6.7m (H-1.2), 5.38s (H-5β; trans), 5.10s (H-5β; cis) PMR (C₆D₆) 12.95s (NH; trans) 10.60s (NH; cis) 8.8d; 8.6d (Ar-H), 7.8m (Ar-H), 7.65d; 7.4d (Ar-H), 6.8m (H-1.2), 5.03s (H-5β; cis), 4.73s (H-5β; trans) isomer ratio trans/cis = 60/40.
MS (C₂₃H₂₃N₅O₇ - 481.45) 481(62) 464(M-17; 18), 447(M-34; 32)

## Claims (Claims for the following Contracting State(s): BE, CH, FR, GB, IT, LI, NL, SE)

1. Novel morphinane skeletoned compounds of formula I and I' the steric isomers thereof 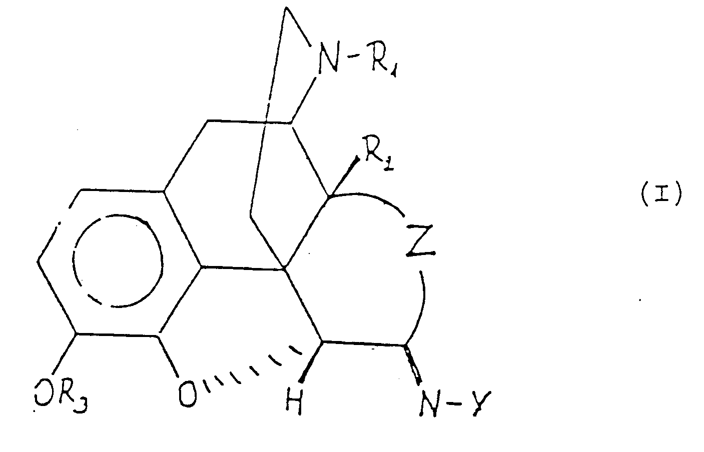 wherein
Z represents-CH₂ -CH₂ or -CH=CH-,
R₁ stands for methyl, -CH₂CH=CH₂, 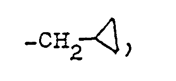
R₂ is hydrogen or hydroxy,
R₃ is hydrogen or methyl,
Y stands for NH₂, -NH-CONH₂, -NH-CSNH₂, -NHC₆H₅, -NH-C₆H₃(NO₂)₂, -NHCOC₆H₅, -NH-SO₂C₆H₄-pCH₃, 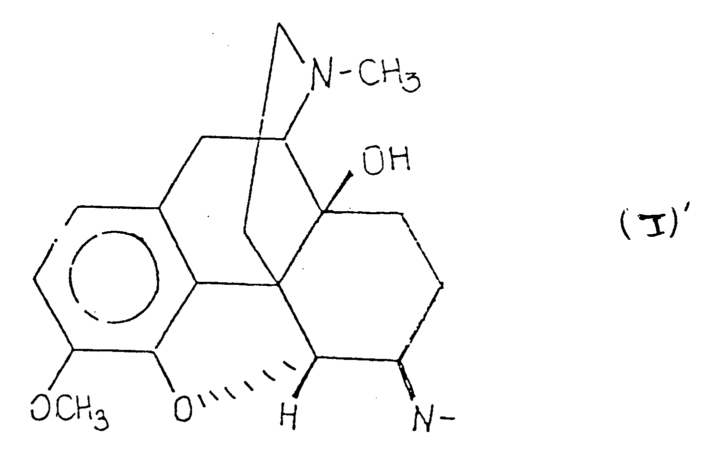
the dotted line represents a bond or hydrogen, and the pharmaceutically acceptable salts thereof with the following provisions applying to the isomers:
when Z represents -CH₂-CH₂-, R₂ represents hydrogen, R₃ represents methyl then Y must be different from -NH-CONH₂, -NHCOC₆H₅, -NH-C₆H₃(NO₂)₂,
when Z represents -CH=CH-, R₂ represents hydrogen and R₃ represents methyl then Y must be different from -NH-CONH₂, -NH-C₆H₃(NO₂)₂, and
when Z represents -CH₂-CH₂- or -CH=CH-, R₂ represents hydroxy, R₃ represents methyl; then Y must be different from -NHC₆H₅,
when Z is -CH₂-CH₂-, R₂ is hydrogen, R₃ is methyl and the dotted line represents a bond, Y must be different from -NH-CONH₂
with the following provisions applying to the isomer mixture:
when Z represents -CH₂-CH₂-, R₂ represents hydrogen, R₃ represents methyl then Y must be different from -NH-CONH₂, -NHCOC₆H₅, -NH-C₆H₃(NO₂)₂,
-NH₂, -NHC₆H₅, when Z represents -CH=CH-, R₂ represents hydrogen and R₃ represents methyl, then Y must be different from -NH-CONH₂, -NH-C₆H₃(NO₂)₂, and
when Z represents -CH=CH-, R₂ represents hydroxy, R₃ represents methyl; then Y must be different from -NHC₆H₅,-NH₂,-NHC₆H₄(NO₂)₂,
when Z represents-CH₂-CH₂-, R₂ represents hydroxy, R₃ represents methyl, then Y must be different from -NHC₆H₅ and -NH₂,
when Z is -CH₂-CH₂-, R₂ is hydroxy, R₃ is hydrogen, the dotted line represents a bond, then Y must be different from -NH₂,
when Z represents -CH₂-CH₂-, R₂ is hydroxy, R₃ is hydrogen, the dotted line represents a bond, then Y must be different from -NH₂,
when R₁ represents methyl or -CH₂-CH=CH₂, R₂ represents hydroxy, R₃ represents hydrogen, Z is -CH₂-CH₂-, the dotted line represents a bond, then Y must be different from -NHC₆H₅,
when R₁ is methyl, R₂ is hydrogen, R₃ is methyl, Z represents -CH=CH-, and the dotted line represents hydrogen, then Y must be different from -NHC₆H₄(NO₂)₂.

2. Steric isomers of a compound selected from the group of
14-OH-dihydrocodeinion hydrazone,
14-OH-dihydrocodeinon azine
Dihydrocodeinone hydrazone
Dihydromorphinone hydrazone
14-OH-codeinon hydrazone
14-OH-dihydrocodeinon semicarbazone
14-OH-codeinon-semicarbazone
14-OH-dihydromorphinone semicarbazone
Dihydromorphinone semicarbazone
Naloxon semicarbazone
Naloxon methylether semicarbazone
14-OH dihydrocodeinone thiosemicarbazone
14-OH-codeinone thiosemicarbazone
Dihydromorphinone thiosemicarbazone
14-OH dihydromorphine thiosemicarbazone
Dihydromorphinone phenylhydrazone
14-OH-dihydromorphinone phenylhydrazone
Dihydrocodeinone-2,4-dinitro-phenylhydrazone
14-OH-dihydrocodeinone-2,4-dinitrophenyl hydrazone
14-OH-codeinon-2,4-dinitrophenyl hydrazone
Dihydromorphinone-2,4-dinitrophenyl hydrazone
14-OH-dihydromorphinone-2,4-dinitrophenyl hydrazone
or the pharmaceutically acceptable salts thereof.

3. A compound selected from the group of
14-OH-dihydrocodeinon azine
Dihydromorphinone hydrazone
14-OH-dihydrocodeinon semicarbazone
14-OH-codeinon-semicarbazone
14-OH-dihydromorphinone semicarbazone
Dihydromorphinone semicarbazone
Naloxon semicarbazone
Naloxon methylether semicarbazone
14-OH dihydrocodeinone thiosemicarbazone
14-OH-codeinone thiosemicarbazone
Dihydromorphinone thiosemicarbazone
14-OH dihydromorphine thiosemicarbazone
Dihydromorphinone phenylhydrazone
14-OH-dihydromorphinone phenylhydrazone
Dihydrocodeinone-2,4-dinitro-phenylhydrazone
14-OH-dihydrocodeinone-2,4-dinitrophenyl hydrazone
Dihydromorphinone-2,4-dinitrophenyl hydrazone
14-OH-dihydromorphinone-2,4-dinitrophenyl hydrazone
and the pharmaceutically acceptable salts thereof.

4. Pharmaceutical composition which comprises at least one compound of the formula (I), the steric isomers or the pharmaceutically acceptable salts thereof in association with at least one excipient.

5. A process for the preparation of the compounds of formula I or steric isomers thereof 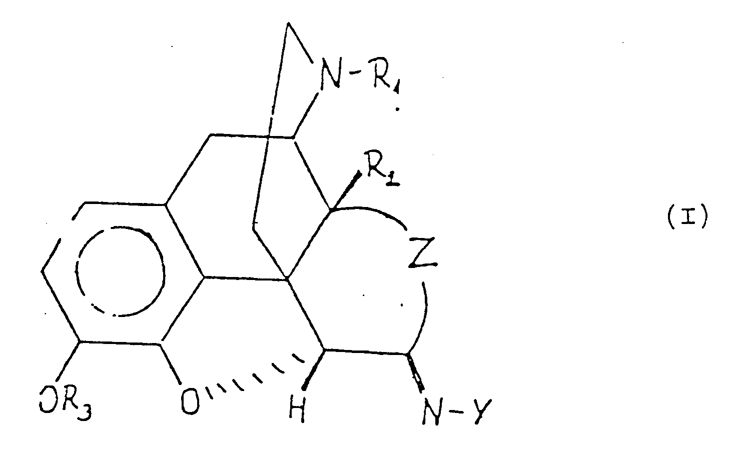 and the pharmaceutically salts thereof, wherein
Z represents CH₂ -CH₂ or -CH=CH-,
R₁ stands for methyl, -CH₂CH=CH₂, 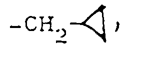
R₂ is hydrogen or hydroxy,
R₃ is hydrogen or methyl,
Y stands for NH₂, -NH-CONH₂, -NH-CSNH₂, -NHC₆H₅, -NH-C₆H₃(NO₂)₂, -NHCOC₆H₅, -NH-SO₂C₆H₄-pCH₃, 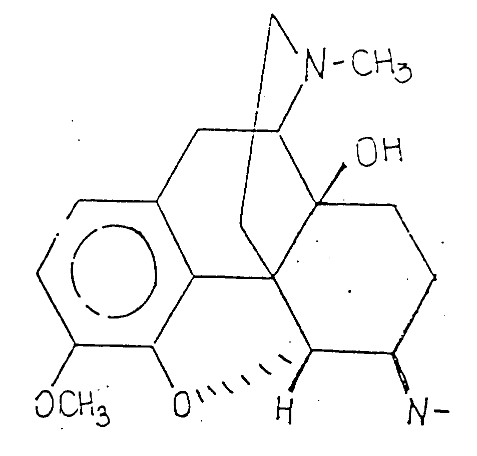
the dotted line represents a bond or hydrogen, with the following provisions applying to the isomers:
when Z represents -CH₂-CH₂-, R₂ represents hydrogen, R₃ represents methyl then Y must be different from -NH-CONH₂, -NHCOC₆H₅, -NH-C₆H₃(NO₂)₂,
when Z represents, -CH=CH-, R₂ represents hydrogen and R₃ represents methyl then Y must be different from -NH-CONH₂, -NH-C₆H₃(NO₂)₂, and
when Z represents -CH₂-CH₂- or -CH=CH-, R₂ represents hydroxy, R₃ represents methyl; then Y must be different from -NHC₆H₅,
when Z is -CH₂-CH₂, R₂ is hydrogen, R₃ is methyl and the dotted line represents a bond, Y must be different from -NH-CONH₂
with the following provisions applying to the isomer mixture :
when Z represents -CH₂-CH₂-, R₂ represents hydrogen, R₃ represents methyl then Y must be different from -NH-CONH₂, -NHCOC₆H₅, -NH-C₆H₃(NO₂)₂, -NH₂, -NHC₆H₅,
when Z represents -CH=CH-, R₂ represents hydrogen and R₃ represents methyl, then Y must be different from -NH-CONH₂, -NH-C₆H₃(NO₂)₂, and
when Z represents -CH=CH-, R₂ represents hydroxy, R₃ represents methyl; then Y must be different from -NHC₆H₅,-NH₂, -NHC₆H₄(NO₂)₂,
when Z represents-CH₂-CH₂-, R₂ represents hydroxy, R₃ represents methyl, then Y must be different from -NHC₆H₅ and -NH₂,
when Z is -CH₂-CH₂-, R₂ is hydroxy, R₃ is hydrogen, the dotted line represents a bond, then Y must be different from -NH₂,
when Z represents -CH=CH-, R₂ is hydroxy, R₃ is hydrogen, the dotted line represents a bond, then Y must be different from -NH₂,
when R₁ represents methyl or -CH₂-CH=CH₂, R₂ represents hydroxy, R₃ represents hydrogen, Z is -CH₂-CH₂-, the dotted line represents a bond, then Y must be different from -NHC₆H₅,
when R₁ is methyl, R₂ is hydrogen, R₃ is methyl, Z represents -CH=CH-, and the dotted line represents hydrogen, then Y must be different from -NHC₆H₄(NO₂)₂. which comprises reacting the morphinane-skeletoned ketones of the formula (II) 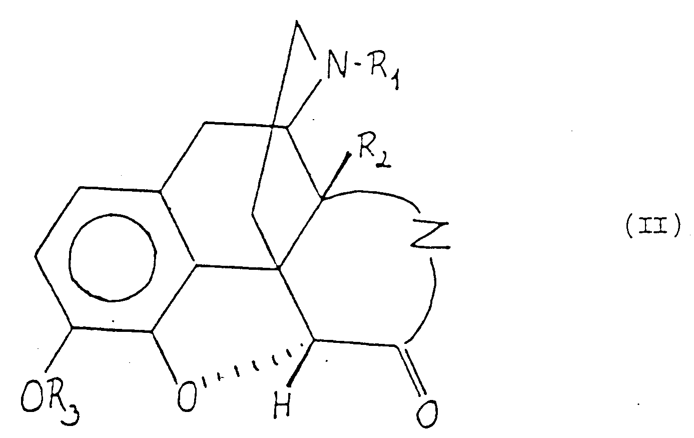 wherein Z, R₁, R₂ and R₃ are the same as defined hereinabove, with a hydrazine of the formula (III)
NH₂Y (III)
wherein Y is the same as defined hereinabove, in aqueous or alcoholic solution and separating the stereoisomers by crystallization and/or chromatography and if desired, transforming the compound thus obtained into a pharmaceutically acceptable salt or liberating the base from the salt thus obtained.

6. The process as claimed in Claim 5 which comprises carrying out the reaction at a temperature of 20-100 ^{o}C.

7. The process as claimed in Claim 5 which comprises using the ketones of the formula (II) and the hydrazones of the formula (III) in a molar ratio of 1:10 if Y=NH₂, while in a molar ratio of 1:15 in all the other cases.

8. The process as claimed in Claim 5, which comprises purifying the crude product of the formula (I) which is the mixture of steric isomers by crystallization and/or chromatography.

9. The process as claimed in Claim 5 which comprises carrying out the reaction of the ketones of the formula (I) and the hydrazines of the formula (II) in methanol or ethanol if Y = -NH₂, -NHCOC₆H₅, -NHSO₂C₆H₄OCH₃, in water or in a mixture of ethanol and water or methanol and water if Y = -NHCONH₂, -NHCSNH₂, while in diluted hydrochloric acid or acetic acid if Y = -NH-C₆H₃(NO₂)₂.

10. A process for the preparation of pharmaceutical compositions which comprises mixing at least one morphinane-skeletoned compound of the formula (I) according to any of claims 1 to 3 and/or the stereoisomers and/or the pharmaceutically acceptable salt thereof with at least one excipient and forming a pharmaceutical formulation.

11. Use of the compounds of any of claims 1 to 3 for the preparation of an analgetic or morphine-antagonistic medicament.

## Claims (Claims for the following Contracting State(s): AT)

1. A process for the preparation of the compounds of formula I and I' or steric isomers thereof 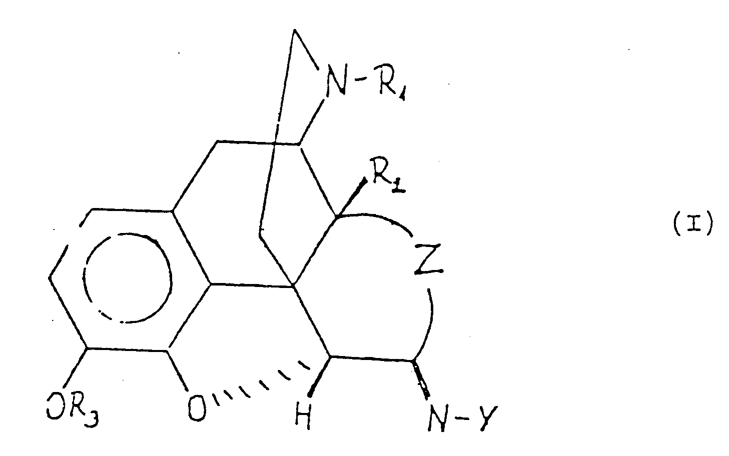 and the pharmaceutically salts thereof, wherein
Z represents CH₂ -CH₂ or -CH=CH-,
R₁ stands for methyl, -CH₂CH=CH₂, 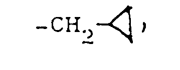
R₂ is hydrogen or hydroxy,
R₃ is hydrogen or methyl,
Y stands for NH₂, -NH-CONH₂, -NH-CSNH₂, -NHC₆H₅, -NH-C₆H₃(NO₂)₂, -NHCOC₆H₅, -NH-SO₂C₆H₄-pCH₃; 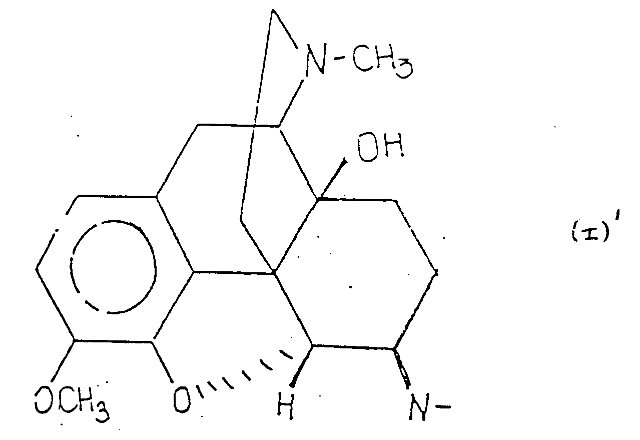
the dotted line represents a bond or hydrogen, with the following provisions applying to the isomers:
when Z represents -CH₂-CH₂-, R₂ represents hydrogen, R₃ represents methyl then Y must be different from -NH-CONH₂, -NHCOC₆H₅, -NH-C₆H₃(NO₂)₂,
when Z represents, -CH=CH-, R₂ represents hydrogen and R₃ represents methyl then Y must be different from -NH-CONH₂, -NH-C₆H₃(NO₂)₂, and
when Z represents -CH₂-CH₂- or -CH=CH-, R₂ represents hydroxy, R₃ represents methyl; then Y must be different from -NHC₆H₅,
when Z is -CH₂-CH₂-, R₂ is hydrogen, R₃ is methyl and the dotted line represents a bond, Y must be different from -NH-CONH₂
with the following provisions applying to the isomer mixture :
when Z represents -CH₂-CH₂-, R₂ represents hydrogen, R₃ represents methyl then Y must be different from -NH-CONH₂, -NHCOC₆H₅, -NH-C₆H₃(NO₂)₂, -NH₂, -NHC₆H₅,
when Z represents -CH=CH-, R₂ represents hydrogen and R₃ represents methyl, then Y must be different from -NH-CONH₂, -NH-C₆H₃(NO₂)₂, and
when Z represents -CH=CH-, R₂ represents hydroxy, R₃ represents methyl; then Y must be different from -NHC₆H₅,-NH₂, -NHC₆H₄(NO₂)₂,
when Z represents-CH₂-CH₂-, R₂ represents hydroxy, R₃ represents methyl, then Y must be different from -NHC₆H₅ and -NH₂,
when Z is -CH₂-CH₂-, R₂ is hydroxy, R₃ is hydrogen, the dotted line represents a bond, then Y must be different from -NH₂,
when Z represents -CH₂-CH₂-, R₂ is hydroxy, R₃ is hydrogen, the dotted line represents a bond, then Y must be different from -NH₂,
when R₁ represents methyl or -CH₂-CH=CH₂, R₂ represents hydroxy, R₃ represents hydrogen, Z is -CH₂-CH₂-, the dotted line represents a bond, then Y must be different from -NHC₆H₅,
when R₁ is methyl, R₂ is hydrogen, R₃ is methyl, Z represents -CH=CH-, and the dotted line represents hydrogen, then Y must be different from -NHC₆H₄(NO₂)₂. which comprises reacting the morphinane-skeletoned ketones of the formula (II) 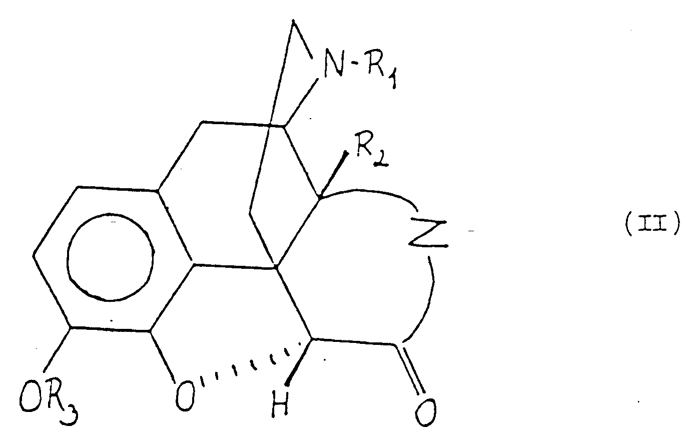 wherein Z, R₁, R₂ and R₃ are the same as defined hereinabove, with a hydrazine of the formula (III)
NH₂Y (III)
wherein Y is the same as defined hereinabove, in aqueous or alcoholic solution and separating the stereoisomers by crystallization and/or chromatography and if desired, transforming the compound thus obtained into a pharmaceutically acceptable salt or liberating the base from the salt thus obtained.

2. The process as claimed in Claim 1 which comprises carrying out the reaction at a temperature of 20-100 ^{o}C.

3. The process as claimed in Claim 1 which comprises using the ketones of the formula (II) and the hydrazones of the formula (III) in a molar ratio of 1:10 if Y=NH₂, while in a molar ratio of 1:15 in all the other cases.

4. The process as claimed in Claim 1, which comprises purifying the crude product of the formula (I) which is the mixture of steric isomers by crystallization and/or chromatography.

5. The process as claimed in Claim 1 which comprises carrying out the reaction of the ketones of the formula (I) and the hydrazines of the formula (II) in methanol or ethanol if Y = -NH₂, -NHCOC₆H₅, -NHSO₂C₆H₄OCH₃, in water or in a mixture of ethanol and water or methanol and water if Y = -NHCONH₂, -NHCSNH₂, while in diluted hydrochloric acid or acetic acid if Y = -NH-C₆H₃(NO₂)₂.

6. A process for the preparation of pharmaceutical compositions which comprises mixing at least one morphinane-skeletoned compound of the formula (I) according to claim 1 and/or the stereoisomers and/or the pharmaceutically acceptable salt thereof with at least one excipient and forming a pharmaceutical formulation.

7. Use of the compounds of formula (I) according to claim 1 for the preparation of an analgetic or morphine-antagonistic medicament.

## Patentansprüche (Patentansprüche für folgende(n) Vertragsstaat(en): BE, CH, FR, GB, IT, LI, NL, SE)

1. Neue Verbindungen mit Morphinan-Gerüst, die durch die Formeln (I) und (I') dargestellt werden, sowie sterische Isomere davon 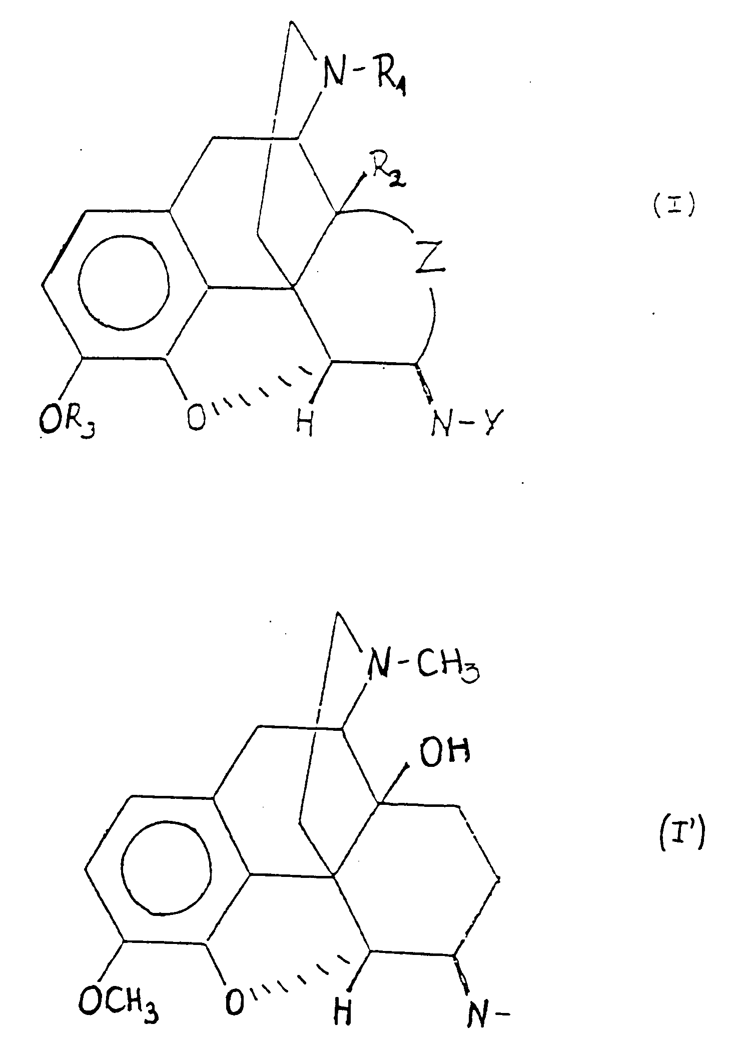 worin
Z -CH₂ -CH₂ oder -CH=CH- darstellt,
R₁ für Methyl, -CH₂CH=CH₂, 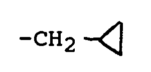 steht,
R₂ Wasserstoff oder Hydroxy ist,
R₃ Wasserstoff oder Methyl ist,
Y für -NH₂, -NH-CONH₂, -NH-CSNH₂, -NHC₆H₅, -NH-C₆H₃(NO₂)₂, -NHCOC₆H₅, -NH-SO₂C₆H₄-pcH₃ steht,
die punktierte Linie eine Bindung oder Wasserstoff darstellt, und die pharmazeutisch akzeptablen Salze davon, wobei für Isomeren die folgenden Maßgaben gelten:
wenn Z -CH₂-CH₂- darstellt, R₂ Wasserstoff, R₃ Methyl darstellt, dann muß Y von -NH-CONH₂, -NHCOC₆H₅, -NH-C₆H₃(NO₂)₂ verschieden sein;
wenn Z -CH=CH- darstellt, R₂ Wasserstoff darstellt und R₃ Methyl darstellt, dann muß Y von -NH-CONH₂, -NH-C₆H₃(NO₂)₂ verschieden sein, und
wenn Z -CH₂-CH₂-oder -CH=CH- darstellt, R₂ Hydroxy darstellt, R₃ Methyl darstellt, dann muß Y von -NHC₆H₅ verschieden sein;
wenn Z -CH₂-CH₂- ist, R₂ Wasserstoff ist, R₃ Methyl ist und die punktierte Linie eine Bindung darstellt, dann muß Y von -NH-CONH₂ verschieden sein;
wobei für das isomere Gemisch die folgenden Maßgaben gelten:
wenn Z -CH₂-CH₂- darstellt, R₂ Wasserstoff darstellt, R₃ Methyl darstellt, dann muß Y von -NH-CONH₂, -NHCOC₆H₅, -NH-C₆H₃(NO₂)₂, -NH₂, -NHC₆H₅ verschieden sein,
wenn Z -CH=CH darstellt, R₂ Wasserstoff und R₃ Methyl darstellt, dann muß Y von -NH-CONH₂,-NHC₆H₃(NO₂)₂ verschieden sein, und
wenn Z -CH=CH darstellt, R₂ Hydroxy darstellt, R₃ Methyl darstellt, dann muß Y von -NHC₆H₅, -NH₂, -NHC₆H₄(NO₂)₂ verschieden sein,
wenn Z -CH₂-CH₂- darstellt, R₂ Hydroxy darstellt, R₃ Methyl darstellt, dann muß Y von -NHC₆H₅ und NH₂ verschieden sein,
wenn Z -CH₂-CH₂ darstellt, R₂ Hydroxy ist, R₃ Wasserstoff ist, die punktierte Linie eine Bindung darstellt, dann muß Y von -NH₂ verschieden sein,
wenn Z -CH₂-CH₂ darstellt, R₂ Hydroxy ist, R₃ Wasserstoff ist, die punktierte Linie eine Bindung darstellt, dann muß Y von -NH₂ verschieden sein,
wenn R₁ Methyl oder -CH₂-CH=CH₂ darstellt, R₂ Hydroxy darstellt, R₃ Wasserstoff darstellt, Z -CH₂-CH₂- ist, die punktierte Linie eine Bindung darstellt, dann muß Y von -NHC₆H₅ verschieden sein,
wenn R₁ Methyl ist, R₂ Wasserstoff ist, R₃ Methyl ist, Z -CH=CH darstellt und die punktierte Linie Wasserstoff darstellt, dann muß Y von -NHC₆H₄(NO₂)₂ verschieden sein.

2. Sterische Isomere einer Verbindung, die aus der Gruppe aus
14-OH-Dihydrocodeinonhydrazon,
14-OH-Dihydrocodeinonazin
Dihydrocodeinonhydrazon
Dihydromorphinonhydrazon
14-OH-Codeinonhydrazon
14-OH-Dihydrocodeinonsemicarbazon
14-OH-Codeinonsemicarbazon
14-OH-Dihydromorphinonsemicarbazon
Dihydromorphinonsemicarbazon
Naloxonsemicarbazon
Naloxonmethylethersemicarbazon
14-OH-Dihydrocodeinonthiosemicarbazon
14-OH-Codeinonthiosemicarbazon
Dihydromorphinonthiosemicarbazon
14-OH-Dihydromorphinthiosemicarbazon
Dihydromorphinonphenylhydrazon
14-OH-Dihydromorphinonphenylhydrazon
Dihydrocodeinon-2,4-dinitro-phenylhydrazon
14-OH-Dihydrocodeinon-2,4-dinitrophenylhydrazon
14-OH-Codeinon-2,4-dinitrophenylhydrazon
Dihydromorphinon-2,4-dinitrophenylhydrazon
14-OH-Dihydromorphinon-2,4-dinitrophenylhydrazon
oder die pharmazeutisch akzeptablen Salze davon.

3. Verbindung, ausgewählt aus der Gruppe
14-OH-Dihydrocodeinonazin
Dihydromorphinohydrazon
14-OH-Dihydrocodeinonsemicarbazon
14-OH-Codeinonsemicarbazon
14-OH-Dihydromorphinonsemicarbazon
Dihydromorphinonsemicarbazon
Naloxonsemicarbazon
Naloxonmethylethersemicarbazon
14-OH-Dihydrocodeinonthiosemicarbazon
14-OH-Codeinonthiosemicarbazon
Dihydromorphinonthiosemicarbazon
14-OH-Dihydromorphinthiosemicarbazon
Dihydromorphinonphenylhydrazon
14-OH-Dihydromorphinonphenylhydrazon
Dihydrocodeinon-2,4-dinitrophenylhydrazon
14-OH-Dihydrocodeinon-2,4-dinitrophenylhydrazon
Dihydromorphinon-2,4-dinitrophenylhydrazon
14-OH-Dihydromorphinon-2,4-dintrophenylhydrazon
und die pharmazeutisch akzeptablen Salze davon.

4. Pharmazeutische Zusammensetzung, welche mindestens eine Verbindung der Formel (I), der sterischen Isomeren oder der pharmazeutisch akzeptablen Salze davon in Verbindung mit mindestens einem Vehikel aufweist.

5. Verfahren zur Herstellung der Verbindungen von Formel (I) oder von sterischen Isomeren davon 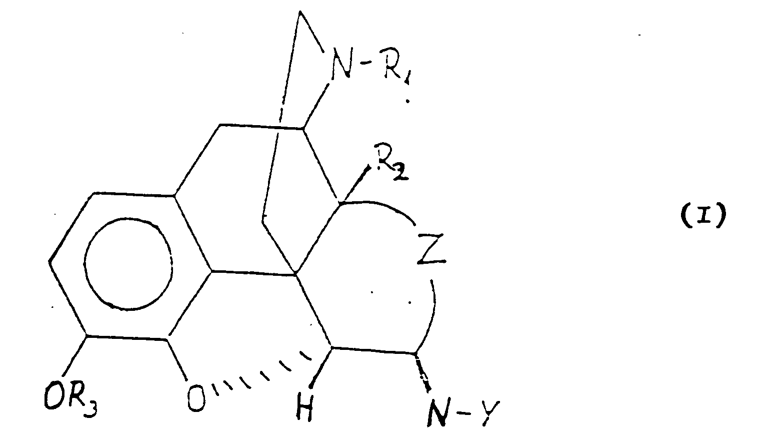 und von pharmazeutisch akzeptablen Salzen davon, worin
Z -CH₂-CH₂ oder -CH=CH- darstellt,
R₁ für Methyl, -CH₂-CH=CH₂, 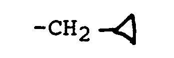 steht,
R₂ Wasserstoff oder Hydroxy ist,
R₃ Wasserstoff oder Methyl ist,
Y für -NH₂, -NH-CONH₂, -NH-CSNH₂, NHC₆H₅, -NH-C₆H₃(NO₂)₂, -NHCOC₆H₅, -NH-SO₂C₆H₄-pCH₃, 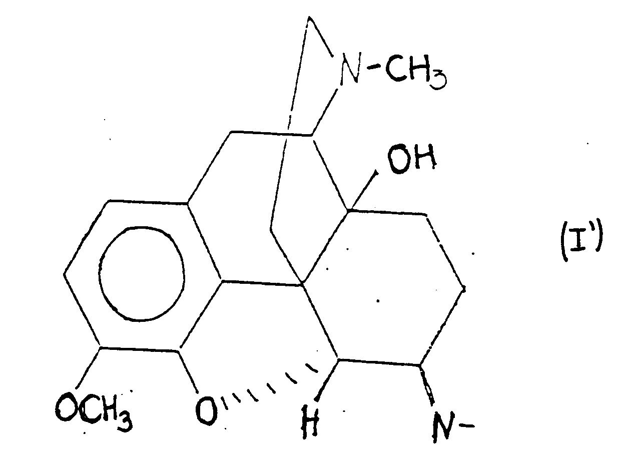
die punktierte Linie eine Bindung oder Wasserstoff darstellt, wobei für die Isomeren folgende Maßgaben gelten:
wenn Z -CH₂-CH₂- darstellt, R₂ Wasserstoff, R₃ Methyl darstellt, dann muß Y von -NH-CONH₂, -NHCOC₆H₅, -NH-C₆H₃(NO₂)₂ verschieden sein;
wenn Z -CH=CH- darstellt, R₂ Wasserstoff darstellt und R₃ Methyl darstellt, dann muß Y von -NH-CONH₂, -NH-C₆H₃(NO₂)₂ verschieden sein, und
wenn Z -CH₂-CH₂-oder -CH=CH- darstellt, R₂ Hydroxy darstellt, R₃ Methyl darstellt, dann muß Y von -NHC₆H₅ verschieden sein;
wenn Z -CH₂-CH₂- ist, R₂ Wasserstoff ist, R₃ Methyl ist und die punktierte Linie eine Bindung darstellt, dann muß Y von - NH-CONH₂ verschieden sein;
wobei für das isomere Gemisch die folgenden Maßgaben gelten:
wenn Z -CH₂-CH₂- darstellt, R₂ Wasserstoff darstellt, R₃ Methyl darstellt, dann muß Y von -NH-CONH₂, -NHCOC₆H₅, -NH-C₆H₃(NO₂)₂, -NH₂, -NHC₆H₅ verschieden sein,
wenn Z -CH=CH darstellt, R₂ Wasserstoff und R₃ Methyl darstellt, dann muß Y von -NH-CONH₂,-NHC₆H₃(NO₂)₂ verschieden sein, und
wenn Z -CH=CH darstellt, R₂ Hydroxy darstellt, R₃ Methyl darstellt, dann muß Y von -NHC₆H₅, -NH₂, -NHC₆H₄(NO₂)₂ verschieden sein,
wenn Z -CH₂-CH₂- darstellt, R₂ Hydroxy darstellt, R₃ Methyl darstellt, dann muß Y von -NHC₆H₅ und NH₂ verschieden sein,
wenn Z -CH₂-CH₂ darstellt, R₂ Hydroxy ist, R₃ Wasserstoff ist, die punktierte Linie eine Bindung darstellt, dann muß Y von -NH₂ verschieden sein,
wenn Z -CH=CH- darstellt, R₂ Hydroxy ist, R₃ Wasserstoff ist, die punktierte Linie eine Bindung darstellt, dann muß Y von -NH₂ verschieden sein,
wenn R₁ Methyl oder -CH₂-CH=CH₂ darstellt, R₂ Hydroxy darstellt, R₃ Wasserstoff darstellt, Z -CH₂-CH₂- ist, die punktierte Linie eine Bindung darstellt, dann muß Y von -NHC₆H₅ verschieden sein,
wenn R₁ Methyl ist, R₂ Wasserstoff ist, R₃ Methyl ist, Z -CH=CH darstellt und die punktierte Linie Wasserstoff darstellt, dann muß Y von -NHC₆H₄(NO₂)₂ verschieden sein,
welches die Reaktion des Ketons der Formel (II) mit Morphinan-Gerüst 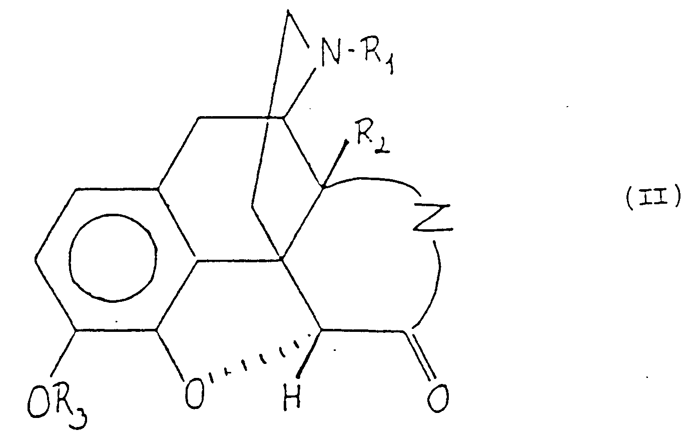 worin Z, R₁, R₂ und R₃ wie oben definiert sind, mit einem Hydrazin der Formel (III)
NH₂Y (III)
worin Y wie oben definiert ist, in wässriger oder alkoholischer Lösung, das Trennen der Stereoisomeren durch Kristallisation und/oder Chromatographie und, wenn gewünscht, das Umwandeln der so erhaltenen Verbindung in ein pharmazeutisch akzeptables Salz oder das Freisetzen der Base aus dem so erhaltenen Salz umfaßt.

6. Verfahren nach Anspruch 5, welches die Durchführung der Reaktion bei einer Temperatur von 20-100°C umfaßt.

7. Verfahren nach Anspruch 5, welches die Verwendung von Ketonen der Formel (II) und Hydrazonen der Formel (III) in einem molaren Verhältnis von 1:10, wenn Y=NH₂, allerdings in einem molaren Verhältnis von 1:15 in allen anderen Fällen umfaßt.

8. Verfahren nach Anspruch 5, welches die Reinigung des Rohproduktes der Formel (I), das ein Gemisch von sterischen Isomeren ist, durch Kristallisation und/oder Chromatographie umfaßt.

9. Verfahren nach Anspruch 5, welches die Durchführung der Reaktion von Ketonen der Formel (I) und Hydrazinen der Formel (II) in Methanol oder Ethanol, wenn Y= -NH₂, -NHCOC₆H₅, -NHSOC₆H₄OCH₃, in Wasser oder in einem Gemisch aus Ethanol und Wasser oder Methanol und Wasser, wenn Y= -NHCONH₂, -NHCSNH₂, hingegen in verdünnter Salzsäure oder Essigsäure, wenn Y= -NH-C₆H₃(NO₂)₂, umfaßt.

10. Verfahren zur Herstellung von pharmazeutischen Zusammensetzungen, das das Mischen mindestens einer Verbindung der Formel (I) mit Morphinan-Gerüst gemäß einem der Ansprüche 1 bis 3 und/oder der Stereoisomeren und/oder der pharmazeutisch akzeptablen Salze davon mit mindestens einem Vehikel sowie die Bildung einer pharmazeutischen Formulation umfaßt.

11. Verwendung der Verbindungen nach einem der Ansprüche 1 bis 3 zur Herstellung eines analgetischen oder Morphin-antagonistischen Medikaments.

## Patentansprüche (Patentansprüche für folgende(n) Vertragsstaat(en): AT)

1. Verfahren zur Herstellung der Verbindungen der Formeln (I) und (I') oder sterischer Isomeren davon 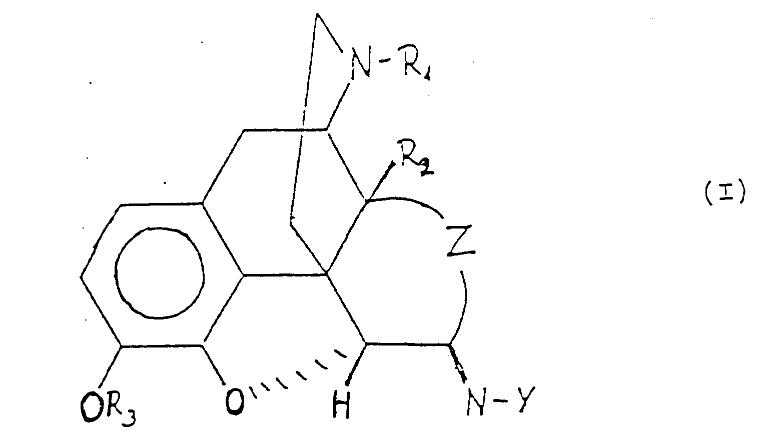 und der pharmazeutisch akzeptablen Salze davon, worin
Z -CH₂-CH₂ oder -CH=CH- darstellt,
R₁ für Methyl, -CH₂-CH=CH₂, 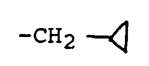 steht,
R₂ Wasserstoff oder Hydroxy ist,
R₃ Wasserstoff oder Methyl ist,
Y für NH₂, -NH-CONH₂, -NH-CSNH₂, -NHC₆H₅, -NH-C₆H₃(NO₂)₂, -NHCOC₆H₅, -NH-SO₂C₆H₄-ₚCH₃ steht; 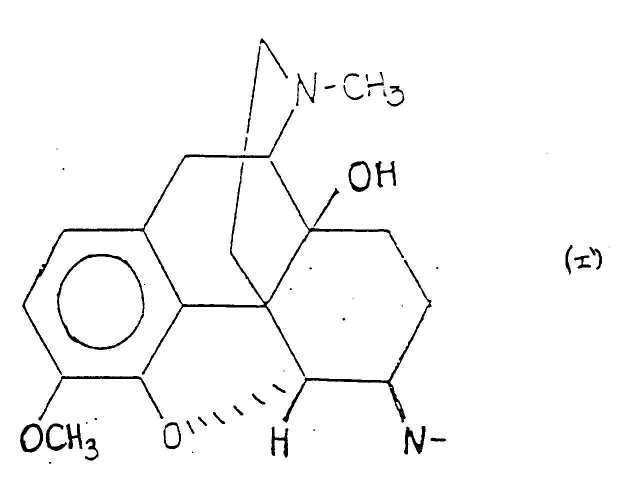
die punktierte Linie eine Bindung oder Wasserstoff darstellt,
wobei für die Isomeren folgende Maßgaben gelten:
wenn Z -CH₂-CH₂- darstellt, R₂ Wasserstoff, R₃ Methyl darstellt, dann muß Y von -NH-CONH₂, -NHCOC₆H₅, -NH-C₆H₃(NO₂)₂ verschieden sein;
wenn Z -CH=CH- darstellt, R₂ Wasserstoff darstellt und R₃ Methyl darstellt, dann muß Y von -NH-CONH₂, -NH-C₆H₃(NO₂)₂ verschieden sein, und
wenn Z -CH₂-CH₂-oder -CH=CH- darstellt, R₂ Hydroxy darstellt, R₃ Methyl darstellt, dann muß Y von -NHC₆H₅ verschieden sein;
wenn Z -CH₂-CH₂- ist, R₂ Wasserstoff ist, R₃ Methyl ist und die punktierte Linie eine Bindung darstellt, dann muß Y von - NH-CONH₂ verschieden sein;
wobei für das isomere Gemisch die folgenden Maßgaben gelten:
wenn Z -CH₂-CH₂- darstellt, R₂ Wasserstoff darstellt, R₃ Methyl darstellt, dann muß Y von -NH-CONH₂, -NHCOC₆H₅, -NH-C₆H₃(NO₂)₂, -NH₂, -NHC₆H₅ verschieden sein,
wenn Z -CH=CH darstellt, R₂ Wasserstoff und R₃ Methyl darstellt, dann muß Y von -NH-CONH₂, -NHC₆H₃(NO₂)₂ verschieden sein, und
wenn Z -CH=CH darstellt, R₂ Hydroxy darstellt, R₃ Methyl darstellt, dann muß Y von -NHC₆H₅, -NH₂, -NHC₆H₄(NO₂)₂ verschieden sein,
wenn Z -CH₂-CH₂- darstellt, R₂ Hydroxy darstellt, R₃ Methyl darstellt, dann muß Y von -NHC₆H₅ und NH₂ verschieden sein,
wenn Z -CH₂-CH₂ darstellt, R₂ Hydroxy ist, R₃ Wasserstoff ist, die punktierte Linie eine Bindung darstellt, dann muß Y von -NH₂ verschieden sein,
wenn Z -CH₂-CH₂ darstellt, R₂ Hydroxy ist, R₃ Wasserstoff ist, die punktierte Linie eine Bindung darstellt, dann muß Y von -NH₂ verschieden sein,
wenn R₁ Methyl oder -CH₂-CH=CH₂ darstellt, R₂ Hydroxy darstellt, R₃ Wasserstoff darstellt, Z -CH₂-CH₂- ist, die punktierte Linie eine Bindung darstellt, dann muß Y von -NHC₆H₅ verschieden sein,
wenn R₁ Methyl ist, R₂ Wasserstoff ist, R₃ Methyl ist, Z - CH=CH darstellt und die punktierte Linie Wasserstoff darstellt, dann muß Y von -NHC₆H₄(NO₂)₂ verschieden sein,
welches die folgenden Schritte umfaßt:
Reaktion von Ketonen der Formel (II), die ein Morphinan-Gerüst aufweisen, 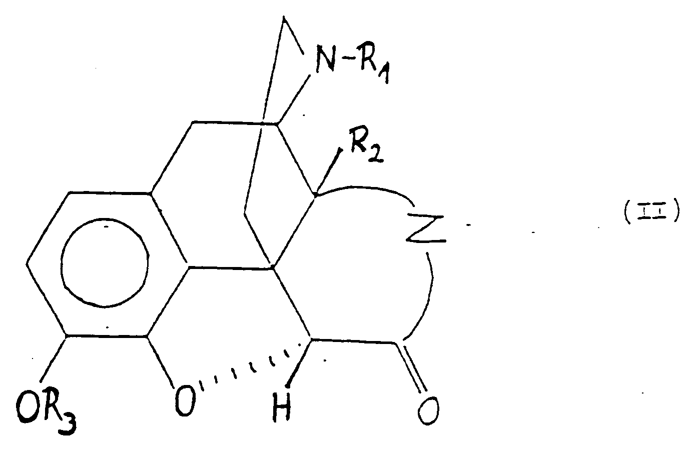 worin Z, R₁, R₂ und R₃ wie oben definiert sind, mit einem Hydrazin der Formel (III)
NH₂Y (III)
worin Y wie oben definiert ist, in wässriger oder alkoholischer Lösung und Trennen der Stereoisomeren durch Kristallisation und/oder Chromatographie und, wenn gewünscht, Überführen der so erhaltenen Verbindung in pharmazeutisch akzeptable Salze oder Freisetzen der Base aus dem so erhaltenen Salz.

2. Verfahren nach Anspruch 1, welches die Durchführung des Verfahrens bei einer Temperatur von 20-100°C umfaßt.

3. Verfahren nach Anspruch 1, welches die Verwendung von Ketonen der Formel (II) und Hydrazonen der Formel (III) in einem molaren Verhältnis von 1:10, wenn Y = NH₂, in allen anderen Fällen hingegen in einem molaren Verhältnis von 1:15, umfaßt.

4. Verfahren nach Anspruch 1, welches die Reinigung des Rohproduktes der Formel (I), das die Mischung von sterischen Isomeren ist, durch Kristallisation und/oder Chromatographie umfaßt.

5. Verfahren nach Anspruch 1, welches die Durchführung der Reaktion von Ketonen der Formel (I) und Hydrazinen der Formel (II) in Methanol oder Ethanol, wenn Y = -NH₂, -NHCOC₆H₅, -NHSOC₆H₄OCH₃, in Wasser oder in einem Gemisch aus Ethanol und Wasser oder Methanol und Wasser, wenn Y = -NHCONH₂, -NHCSNH₂, hingegen in verdünnter Salzsäure oder Essigsäure, wenn Y = -NH-C₆H₃(NO₂)₂, umfaßt.

6. Verfahren zur Herstellung von pharmazeutischen Zusammensetzungen, das das Mischen mindestens einer Verbindung der Formel (I) mit Morphinan-Gerüst gemäß Anspruch 1 und/oder der Stereoisomeren und/oder der pharmazeutisch akzeptablen Salze davon mit mindestens einem Vehikel sowie die Bildung einer pharmazeutischen Formulation umfaßt.

7. Verwendung der Verbindungen der Formel (I) nach Anspruch 1 zur Herstellung eines analgetischen oder Morphin-antagonistischen Medikaments.

## Revendications (Revendications pour l'(les) Etat(s) contractant(s) suivant(s): BE, CH, FR, GB, IT, LI, NL, SE)

1. Nouveaux composés à squelette morphinane de formule (I) et (I') ou leurs isomères stériques : 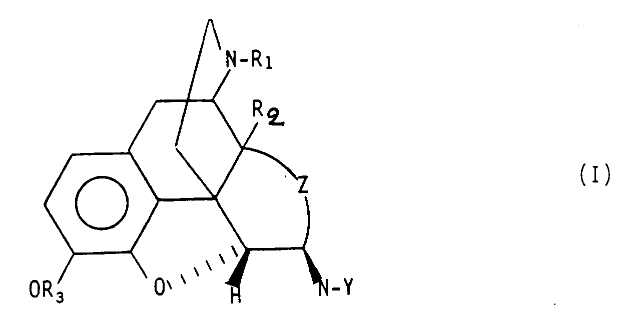 dans laquelle :
Z représente -CH₂-CH₂- ou -CH=CH-,
R₁ représente le méthyle, -CH₂CH=CH₂, 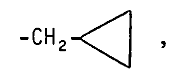
R₂ est un hydrogène ou hydroxy,
R₃ est un hydrogène ou méthyle,
Y représente NH₂, -NH-CONH₂, -NH-CSNH₂, -NHC₆ H₅, -NH-C₆H₃(NO₂)₂, -NHCOC₆H₅, -NH-SO₂C₆H₄-pCH₃, 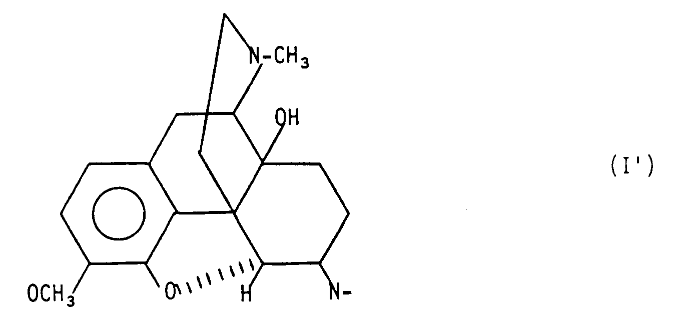
la ligne interrompue représente une liaison ou un hydrogène et leurs sels pharmaceutiquement acceptables,
avec les conditions suivantes concernant les isomères :
quand Z représente -CH₂-CH₂-, R₂ représente l'hydrogène R₃ représente le méthyle alors il faut que Y soit différent de -NH-CONH₂, -NHCOC₆H₅, -NH-C₆H₃(NO₂)₂,
quand Z représente -CH=CH-, R₂ représente l'hydrogène et R₃ représente le méthyle alors il faut que Y soit différent de -NH-CONH₂, -NH-C₆H₃(NO₂)₂, et
quand Z représente -CH₂-CH₂- ou -CH=CH-, R₂ représente un hydroxy, R₃ représente le méthyle, alors il faut que Y soit différent de -NHC₆H₅,
quand Z est -CH₂-CH₂-, R₂ est l'hydrogène, R₃ est le méthyle et la ligne interrompue représente une liaison, il faut que Y soit différent de -NH-CONH₂,
avec les conditions suivantes qui s'appliquent au mélange d'isomères : quand Z représente -CH₂-CH₂-, R₂ représente l'hydrogène, R₃ représente le méthyle alors il faut que Y soit différent de -NH-CONH₂, -NHCOC₆H₅, -NH-C₆H₃(NO₂)₂, -NH₂, -NHC₆H₅,
quand Z représente -CH=CH-, R₂ représente l'hydrogène et R₃ représente le méthyle, alors il faut que Y soit différent de -NH-CONH₂, -NH-C₆H₃(NO₂)₂, et
quand Z représente -CH=CH-, R₂ représente l'hydroxy, R₃ représente le méthyle, alors il faut que Y soit différent de -NHC₆H₅, -NH₂, -NHC₆H₄(NO₂)₂,
quand Z représente -CH₂-CH₂-, R₂ représente l'hydroxy, R₃ représente le méthyle, alors il faut que Y soit différent de -NHC₆H₅ et -NH₂, quand Z est -CH₂-CH₂-, R₂ est l'hydroxy, R₃ est l'hydrogène, la ligne interrompue représente une liaison, alors il faut que Y soit différent de -NH₂,
quand Z représente -CH₂-CH₂-, R₂ est l'hydroxy, R₃ est l'hydrogène, la ligne interrompue représente une liaison, alors il faut que Y soit différent de -NH₂,
quand R₁ représente le méthyle ou -CH₂-CH=CH₂-, R₂ représente l'hydroxy, R₃ représente l'hydrogène, Z est -CH₂-CH₂-, la ligne interrompue représente une liaison, alors il faut que Y soit différent de -NHC₆H₅,
quand R₁ est le méthyle, R₂ est l'hydrogène, R₃ est le méthyle, Z représente -CH=CH-, et la ligne interrompue représente l'hydrogène, alors il faut que Y soit différent de-NHC₆H₄(NO₂)₂.

2. Isomères stériques d'un composé choisi dans le groupe de :
14-OH-dihydrocodéinone hydrazone
14-OH-dihydrocodéinone azine
Dihydrocodéinone hydrazone
Dihydromorphinone hydrazone
14-OH-codéinone hydrazone
14-OH-dihydrocodéinone semicarbazone
14-OH-codéinone-semicarbazone
14-OH-dihydromorphinone semicarbazone
Dihydromorphinone semicarbazone
Naloxon semicarbazone
Naloxon méthyléther semicarbazone
14-OH dihydrocodéinone thiosemicarbazone
14-OH-codéinone thiosemicarbazone
Dihydromorphinone thiosemicarbazone
14-OH dihydromorphine thiosemicarbazone
Dihydromorphinone phénylhydrazone
14-OH-dihydromorphinone phénylhydrazone
Dihydrocodéinone-2,4-dinitro-phénylhydrazone
14-OH-dihydrocodéinone-2,4-dinitrophényl hydrazone
14-OH-codéinone-2,4-dinitrophényl hydrazone
Dihydromorphinone-2,4-dinitrophényl hydrazone
14-OH-dihydromorphinone-2,4-dinitrophényl hydrazone
ou leurs sels pharmaceutiquement acceptables.

3. Composé choisi dans le groupe de :
14-OH-dihydrocodéinone azine
Dihydromorphinone hydrazone
14-OH-dihydrocodéinone semicarbazone
14-OH-codéinone-semicarbazone
14-OH-dihydromorphinone semicarbazone
Dihydromorphinone semicarbazone
Naloxon semicarbazone
Naloxon méthyléther semicarbazone
14-OH dihydrocodéinone thiosemicarbazone
14-OH-codéinone thiosemicarbazone
Dihydromorphinone thiosemicarbazone
14-OH dihydromorphine thiosemicarbazone
Dihydromorphinone phénylhydrazone
14-OH-dihydromorphinone phénylhydrazone
Dihydrocodéinone-2,4-dinitro-phénylhydrazone
14-OH-dihydrocodéinone-2,4-dinitrophényl hydrazone
Dihydromorphinone-2,4-dinitrophényl hydrazone
14-OH-dihydromorphinone-2,4-dinitrophényl hydrazone
et leurs sels pharmaceutiquement acceptables.

4. Composition pharmaceutique qui comprend au moins un composé de formule (I), les isomères stériques ou leurs sels pharmaceutiquement acceptables en association avec au moins un excipient.

5. Procédé de préparation des composés de formule (I) ou leurs isomères stériques 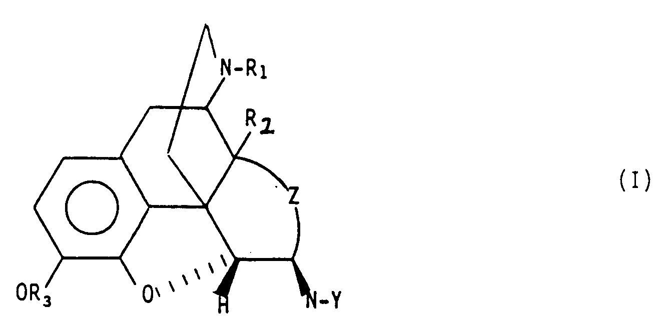 et leurs sels pharmaceutiquement acceptables, dans laquelle :
Z représente -CH₂-CH₂ ou -CH=CH-,
R₁ représente le méthyle, -CH₂CH=CH₂, 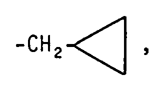
R₂ est un hydrogène ou hydroxy,
R₃ est un hydrogène ou méthyle,
Y représente NH₂, -NH-CONH₂, -NH-CSNH₂, -NHC₆H₅, -NH-C₆H₃(NO₂)₂, -NHCOC₆H₅, -NH-SO₂C₆H₄-pCH₃, 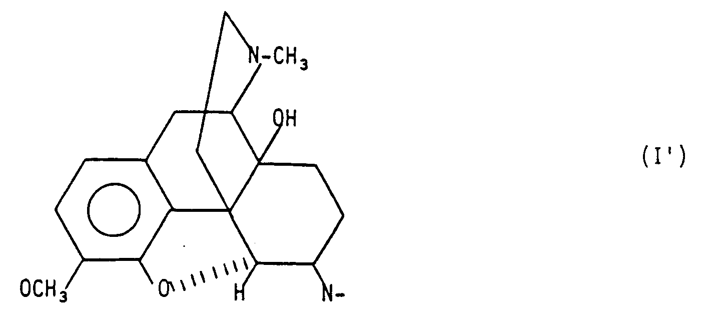
la ligne interrompue représente une liaison ou un hydrogène, avec les conditions suivantes concernant les isomères : quand Z représente -CH₂-CH₂-, R₂ représente l'hydrogène, R₃ représente le méthyle alors il faut que Y soit différent de -NH-CONH₂, -NHCOC₆H₅, -NH-C₆H₃(NO₂)₂,
quand Z représente -CH=CH-, R₂ représente l'hydrogène et R₃ représente le méthyle alors il faut que Y soit différent de -NH-CONH₂, -NH-C₆H₃(NO₂)₂, et
quand Z représente -CH₂-CH₂- ou -CH=CH-, R₂ représente un hydroxy, R₃ représente le méthyle, alors il faut que Y soit différent de -NHC₆H₅
quand Z est -CH₂-CH₂-, R₂ est l'hydrogène, R₃ est le méthyle et la ligne interrompue représente une liaison, il faut que Y soit différent de -NH-CONH₂,
avec les conditions suivantes qui s'appliquent au mélange d'isomères :
quand Z représente -CH₂-CH₂-, R₂ représente l'hydrogène, R₃ représente le méthyle alors il faut que Y soit différent de -NH-CONH₂, -NHCOC₆H₅, -NH-C₆H₃(NO₂)₂, -NH₂, -NHC₆H₅,
quand Z représente -CH=CH-, R₂ représente l'hydrogène et R₃ représente le méthyle, alors il faut que Y soit différent de -NH-CONH₂, -NH-C₆H₃(NO₂)₂, et
quand Z représente -CH=CH-, R₂ représente un hydroxy, R₃ représente le méthyle, alors il faut que Y soit différent de -NHC₆H₅ -NH₂, -NHC₆H₄(NO₂)₂,
quand Z représente -CH₂-CH₂-, R₂ représente l'hydroxy, R₃ représente le méthyle, alors il faut que Y soit diffférent de -NHC₆H₅ et -NH₂,
quand Z est -CH₂-CH₂-, R₂ est l'hydroxy, R₃ est l'hydrogène, la ligne interrompue représente une liaison, alors il faut que Y soit différent de -NH₂,
quand Z représente -CH =CH -, R₂ est l'hydroxy, R₃ est l'hydrogène, la ligne interrompue représente une liaison, alors il faut que Y soit différent de -NH₂,
quand R₁ représente le méthyle ou -CH₂-CH=CH₂-, R₂ représente l'hydroxy, R₃ représente l'hydrogène, Z est -CH₂-CH₂-, la ligne interrompue représente une liaison, alors il faut que Y soit différent de -NHC₆H₅,
quand R₁ est le méthyle,R₂ est l'hydrogène, R₃ est le méthyle, Z représente -CH=CH-, et la ligne interrompue représente l'hydrogène, alors il faut que Y soit différent de NHC₆H₄(NO₂)₂,
qui comprend la réaction des cétones à squelette morphinane de la formule (II) : 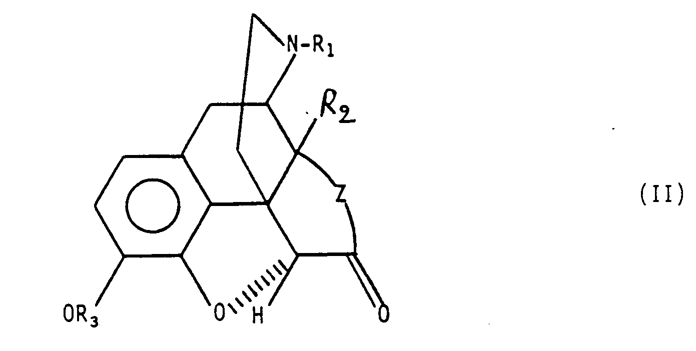 dans laquelle Z, R₁, R₂ et R₃ sont tels que définis ci-dessus, avec une hydrazine de formule (III) :
NH₂Y (III)
dans laquelle Y est tel que défini ci-dessus, en solution aqueuse ou alcoolique, et de séparer les stéréoisomères par cristallisation et/ou chromatographie et, si c'est souhaité, de transformer le composé ainsi obtenu en un sel pharmaceutiquement acceptable ou en libérant la base à partir du sel ainsi obtenu.

6. Procédé selon la revendication 5 qui comprend la réalisation de la réaction à une température de 20-100°C.

7. Procédé selon la revendication 5 qui comprend l'utilisation des cétones de formule (II) et les hydrazones de formule (III) en proportion molaire de 1:10 si Y = NH₂, et dans une proportion molaire de 1:15 dans tous les autres cas.

8. Procédé selon la revendication 5, qui comprend la purification du produit brut de formule (I) qui est le mélange d'isomères stériques par cristallisation et/ou chromatographie.

9. Procédé selon la revendication 5, qui comprend la réalisation de la réaction des cétones de formule (I) et des hydrazines de formule (II) dans le méthanol ou l'éthanol si Y = -NH₂, -NHCOC₆H₅, -NHSO₂C₆H₄OCH₃, dans l'eau ou dans un mélange d'éthanol et d'eau ou de méthanol et d'eau si Y = -NHCONH₂, -NHCSNH₂, ou dans l'acide chlorhydrique dilué ou dans l'acide acétique si Y = -NH-C₆H₃(NO₂)₂.

10. Procédé de préparation de compositions pharmaceutiques qui comprend le mélange d'au moins un composé à squelette morphinane de formule (I) selon l'une quelconque des revendications 1 à 3 et/ou les stéréoisomères, et/ou leurs sels pharmaceutiquement acceptables avec au moins un excipient et de réaliser une formulation pharmaceutique.

11. Utilisation de composés selon l'une quelconque des revendications 1 à 3 pour préparer un médicament analgésique ou morphine-antagoniste.

## Revendications (Revendications pour l'(les) Etat(s) contractant(s) suivant(s): AT)

1. Procédé de préparation des composés de formule I et I' ou leurs isomères stériques : 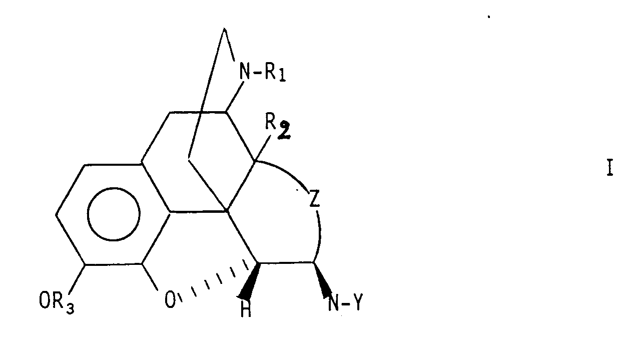 et leurs sels pharmaceutiquement acceptables, dans laquelle
Z représente -CH₂-CH₂ ou -CH=CH-,
R₁ représente le méthyle, -CH₂CH=CH₂, 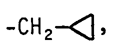
R₂ est un hydrogène ou hydroxy,
R₃ est un hydrogène ou méthyle,
Y représente NH₂, -NH-CONH₂, -NH-CSNH₂, -NHC₆H₅, -NH-C₆H₃(NO₂)₂, -NHCOC₆H₅, -NH-SO₂C₆H₄-pCH₃, 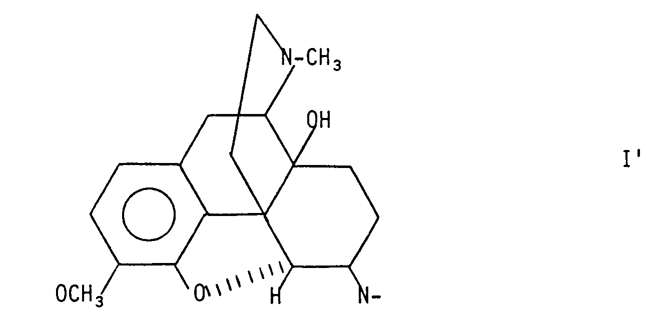
la ligne interrompue représente une liaison ou un hydrogène, avec les conditions suivantes concernant les isomères :
quand Z représente -CH₂-CH₂-, R₂ représente l'hydrogène, R₃ représente le méthyle alors il faut que Y soit différent de -NH-CONH₂, -NHCOC₆H₅, -NH-C₆H₃(NO₂)₂,
quand Z représente -CH=CH-, R₂ représente l'hydrogène et R₃ représente le méthyle alors il faut que Y soit différent de -NH-CONH₂, -NH-C₆H₃(NO₂)₂, et
quand Z représente -CH₂-CH₂- ou -CH=CH-, R₂ représente un hydroxy, R₃ représente le méthyle, alors il faut que Y soit différent de -NHC₆H₅,
Quand Z est -CH₂-CH₂-, R₂ est l'hydrogène, R₃ est le méthyle et la ligne interrompue représente une liaison, il faut que Y soit différent de -NH-CONH₂,
avec les conditions suivantes qui s'appliquent au mélange d'isomères :
quand Z représente -CH₂-CH₂-, R₂ représente l'hydrogène, R₃ représente le méthyle alors il faut que Y soit différent de -NH-CONH₂, -NHCOC₆H₅, -NH-C₆H₃(NO₂)₂, -NH₂, -NHC₆H₅,
quand Z représente -CH=CH-, R₂ représente l'hydrogène et R₃ représente le méthyle, alors il faut que Y soit différent de -NH-CONH₂, -NH-C₆H₃(NO₂)₂, et
quand Z représente -CH=CH-, R₂ représente un hydroxy, R₃ représente le méthyle, alors il faut que Y soit différent de -NHC₆H₅, NH₂, -NHC₆H₄(NO₂)₂,
quand Z représente -CH₂-CH₂-, R₂ représente l'hydroxy, R₃ représente le méthyle, alors il faut que Y soit différent de -NHC₆H₅ et -NH₂,
quand Z est -CH₂-CH₂-, R₂ est l'hydroxy, R₃ est l'hydrogène, la ligne interrompue représente une liaison, alors il faut que Y soit différent de NH₂,
quand Z représente -CH₂-CH₂-, R₂ est l'hydroxy, R₃ est l'hydrogène, la ligne interrompue représente une liaison, alors il faut que Y soit différent de -NH₂,
quand R₁ représente le méthyle ou -CH₂-CH=CH₂-, R₂ représente l'hydroxy, R₃ représente l'hydrogène, Z est -CH₂-CH₂-, la ligne interrompue représente une liaison, alors il faut que Y soit différent de -NHC₆H₅,
quand R₁ est le méthyle, R₂ est l'hydrogène, R₃ est le méthyle, Z représente -CH=CH-, et la ligne interrompue représente l'hydrogène, alors il faut que Y soit différent de-NHC₆H₄(NO₂)₂,
qui comprend la réaction des cétones à squelette morphinane de la formule II : 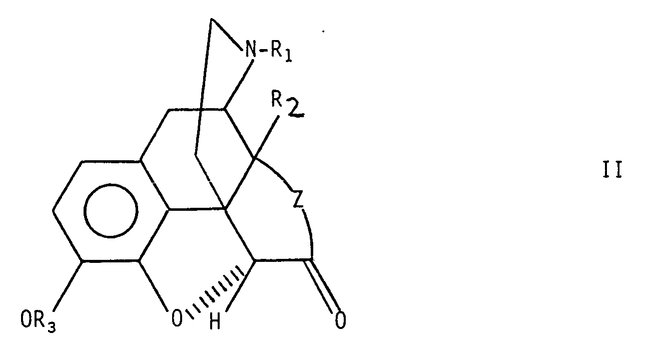 dans laquelle Z, R₁, R₂ et R₃ sont tels que définis ci-dessus, avec une hydrazine de formule III :
NH₂Y III
dans laquelle Y est tel que défini ci-dessus, en solution aqueuse ou alcoolique, et de séparer les stéréoisomères par cristallisation et/ou chromatographie et, si c'est souhaité, de transformer le composé ainsi obtenu en un sel pharmaceutiquement acceptable ou en libérant la base à partir du sel ainsi obtenu.

2. Procédé selon la revendication 1, qui comprend la réalisation de la réaction à une température de 20-100°C.

3. Procédé selon la revendication 1, qui comprend l'utilisation des cétones de formule II et les hydozones de formule III en proportion molaire de 1:10 si Y = NH₂, et dans une proportion molaire de 1:15 dans tous les autres cas.

4. Procédé selon la revendication 1, qui comprend la purification du produit brut de formule I qui est le mélange d'isomères stériques par cristallisation et/ou chromatographie.

5. Procédé selon la revendication 1, qui comprend la réalisation de la réaction de cétones de formule I et des hydrazines de formule II dans le méthanol ou l'éthanol si Y = -NH₂ , -NHCOC₆H₅, -NHSO₂C₆H₄OCH₃, dans l'eau ou dans un mélange d'éthanol et d'eau ou de méthanol et d'eau si Y = -NHCONH₂, -NHCSNH₂, ou dans l'acide chlorhydrique dilué ou dans l'acide acétique si Y = -NH-C₆H₃(NO₂)₂.

6. Procédé de préparation de compositions pharmaceutiques qui comprend le mélange d'au moins un composé à squelette morphinane de formule I selon la revendication 1 et/ou les stéréoisomères, et/ou leurs sels pharmaceutiquement acceptables avec au moins un excipient et de réaliser une formulation pharmaceutique.

7. Utilisation de composés de formule I selon la revendication 1, pour préparer un médicament analgésique ou morphine-antagoniste.
